# EUROPÄISCHE PATENTSCHRIFT

(11) **EP 2 247 709 B1**
(45) Veröffentlichungstag und Bekanntmachung des Hinweises auf die Patenterteilung: **04.07.2012**
(21) Anmeldenummer: 09715339.9
(22) Anmeldetag: 19.02.2009
(51) Int. Cl.: C12M 1/00, B01F 11/00

(54) **SELBSTREINIGENDES REAKTORSYSTEM MIT DREHBAREM REAKTORGEFÄSS**
SELF-CLEANING REACTOR SYSTEM COMPRISING A ROTATABLE REACTOR VESSEL
SYSTÈME DE RÉACTEUR AUTONETTOYANT COMPORTANT UNE CUVE DE RÉACTEUR ROTATIVE

(30) Priorität: 26.02.2008 DE 102008011214
(43) Veröffentlichungstag der Anmeldung: 10.11.2010
(73) Patentinhaber: Baumgardt, Jens, 14169 Berlin (DE)
(72) Erfinder: Baumgardt, Jens, 14169 Berlin (DE)
(74) Vertreter: Morawski, Birgit
(86) Internationale Anmeldenummer: PCT/EP2009/051972
(87) Internationale Veröffentlichungsnummer: WO 2009/106475

(56) Entgegenhaltungen:
- EP-A1- 0 346 990
- EP-A1- 1 661 979
- WO-A1-02/42409
- WO-A2-97/00002
- FR-A5- 2 166 590
- JP-A- 6 022 745
- JP-A- 2003 009 852
- US-A- 3 847 749
- US-A- 4 264 741

## Beschreibung

Die vorliegende Erfindung betrifft ein Reaktorsystem mit einem drehbaren Reaktorgefäß nach dem Oberbegriff des Anspruchs 1, eine dem Reaktorgefäß angeordnete Materialträgereinheit nach Anspruch 16, die Verwendung des Reaktorsystems nach Anspruch 18 sowie ein Verfahren zur Herstellung von Produkten nach Anspruch 20.

Die Herstellung und Bearbeitung von Material, insbesondere biologischem Material, kann über einen längeren Zeitraum in Form von sog. Batchverfahren oder in kontinuierlichen Verfahren erfolgen.

Bei der Langzeitkultivierung von Zellen wird dabei z.B. zwischen seriellen und kontinuierlichen Kulturmethoden unterschieden.

Aus Lenski, R.E. und Travisiano, M. (1994): Dynamics of adaptation and diversification: A 10,000- generation experiment with bacterial populations, Proc. Natl.Acad.Sci.USA 91 6808-6814 ist ein Verfahren zur seriellen Langzeitkultivierung von Zellen bekannt. Innerhalb der seriellen Kulturmethode werden Reaktorgefäße mit sterilem Nährmedium mit Zellen beimpft. Die Zellen werden vorher unter identischen Wachstumsbedingungen angezogen. Nachdem die Zellen zu einer hohen Dichte gewachsen sind, wird eine Fraktion dieser Zellen entnommen und ein neues Reaktorgefäß beimpft. Der Zyklus wird wiederholt. Mit dieser Methode wurden die längsten Kultivierungszeiten erreicht.

Aus Dijkhuizen, D.E. (1993): "Chemostats used for studying natural selection and adaptive evolution", Meth.Enzymol.224, 613-631 ist eine kontinuierliche Kulturmethode bekannt. Bei dem bekannten Verfahren wird ein Reaktorgefäß kontinuierlich mit Nährmedium versorgt. Die Zellen befinden sich in einer andauernden Wachstumsphase, die direkt von der Menge des zugeleiteten Nährmediums abhängig ist. Die Experimente, die mit dieser Methode durchgeführt wurden, mussten nach kurzer Zeit wieder abgebrochen werden. Die Dauer der Kultivierung bei herkömmlichen Verfahren ist begrenzt, da mit der Zeit die Wahrscheinlichkeit von Kontaminationen steigt. Solche Kontaminationen sind beispielsweise bakterielle Infektionen. Ablagerungen aus proteinreichen Nährmedien und abgestorbenes Zellmaterial blockieren die Leitungen. Sie reichem sich auf den Innenseiten der Reaktoren und Leitungen an. Die Nährstoffversorgung der Kultur wird dann ungleichmäßig. Die Nährstoffversorgung im Reaktor verschlechtert sich durch die Akkumulation von überflüssigem Substrat. Weiterhin wachsen die kultivierten Zellen mit zunehmender Zeit nicht nur im Reaktorgefäß, sondern auch in den zu- und ableitenden Leitungsabschnitten (Rückwachstum). Alle Mängel führen zu einer mangelhaften Versorgung der Zellen. Darauf folgt der Untergang der Kultur. Das Experiment muss in der Regel nach maximal fünf bis sechs Wochen abgebrochen werden.

JP2003-009852 offenbart eine Vorrichtung zur Kultivierung von multipotenten Stammzellen. Diese Vorrichtung besteht aus einem Reaktorgefäß das in einem inneren Rahmen starr befestigt ist. Der innere Rahmen wird über einen Motor angetrieben, so dass eine Rotation des Reaktorgefäßes um seine x-Achse erfolgt. Der innere Rahmen ist seinerseits an einem äußeren Rahmen befestigt, der wiederum von einem Motor angetrieben wird und eine Rotation des Reatorgefäßes in vertikaler Richtung ermöglicht. Eine Rotation des Reaktorgefäßes um seine drei räumlichen Achsen ist nicht offenbart.

Bekannte Vorrichtungen ermöglichen adhärentes Zellwachstum auf Trägermaterialien oder den Innenoberflächen der Kulturapparatur.

Die vorstehend beschriebenen Techniken haben den Nachteil, dass die Kultivierung von adhärenten Zellen über lange Zeiträume unter kontrollierten Bedingungen nicht gewährleistet ist.

Ein Biofilm setzt sich aus Milliarden von dicht aneinander haftenden Zellen zusammen. Jede dieser Zellen besitzt eine einzigartige genetische Ausstattung, durch die sie sich an die herrschenden Umweltbedingungen anpassen kann. Jede Zelle kann Erbgut der Nachbarzellen aufnehmen und in ihr eigenes Erbgut einbauen. Ändern sich die Umweltbedingungen, beginnen die Zellen untereinander ihre Gene auszutauschen, bis eine Ausstattung gefunden ist, die optimal an die neuen Bedingungen angepasst ist. Im Gegensatz zu einzelnen, frei schwimmenden Zellen stellt ein Biofilm ein so genanntes Supragenom dar, aus dem ein unerschöpflicher Vorrat genetischer Anpassungen generiert werden kann. Biofilme sind somit robuster und effektiver im Austausch ihrer Gene als die in herkömmlichen Reaktoren verwendeten, frei schwimmenden Zellen (Ehrlich et al: 2006: Bacterial Plurality as a General Mechanism Driving Persistence in Chronic Infections. Clin. Orth. Relat. Res. 584, p.31-35).

Der vorliegenden Erfindung lag daher das Problem zugrunde, ein Reaktorsystem zur Verfügung zu stellen, welches die kontinuierliche Herstellung und/oder Bearbeitung von Materialien in einem geschlossen System ermöglicht, ohne dass das Verfahren z.B. zum Zwecke der Reinigung des Gefäßes unterbrochen werden muss.

Diese Aufgabe wird erfindungsgemäß durch ein Reaktorsystem mit den Merkmalen des Anspruchs 1 gelöst.

Danach umfasst das erfindungsgemäße Reaktorsystem mindestens ein Reaktorgefäß zur Herstellung und/oder Behandlung eines Materials, insbesondere zur kontinuierlichen Kultivierung von biologischem Material. Das Reaktorgefäß ist um mindestens drei seiner räumlichen Achsen (x,y, z)-Achsen drehbar angeordnet, wobei das Reaktorgefäß an mindestens drei Chassis (1,2,24) gekoppelt ist und weist mindestens eine innerhalb des Reaktorgefäßes angeordnete Materialträgereinheit auf Somit kann eine horizontale Rotation des Reaktorgefäßes um die x-Achse und eine vertikale Rotation des Reaktorgefäßes um die y-Achse erfolgen.

Das Reaktorgefäß ist um seine drei räumlichen (x, y, z)-Achsen drehbar angeordnet, wodurch eine zusätzliche Drehung des Reaktorgefäßes in der z-Ebene möglich wird.

Das Reaktorgefäß ist an mindestens drei Chassis gekoppelt. Die Kopplung des Reaktorgefäßes mit den Chassis oder Gestellen ermöglicht die Rotation und Bewegung des Reaktorgefäßes in den räumlichen Ebenen.

Dabei ist das Reaktorgefäß an ein erstes Chassis gekoppelt, welches eine Rotationsachse um die horizontale x-Achse aufweist. Das erste Chassis ermöglicht somit eine Rotation des Reaktorgefäßes um seine horizontale x-Achse.

Das Reaktorgefäß ist ebenfalls an ein zweites Chassis gekoppelt, welches eine Rotationsachse um die vertikale y-Achse aufweist. Das zweite Chassis ermöglicht somit eine Rotation des Reaktorgefäßes um seine vertikale y-Achse.

Das erste und zweite Chassis werden bevorzugt mit mindestens einem Schrittmotor, insbesondere zwei oder drei Schrittmotoren angetrieben.

Auch ist das Reaktorgefäß an ein drittes Chassis gekoppelt, welches eine Rotation der Reaktorgefäßanordnung umfassend Reaktorgefäß mit ersten und zweiten Chassis in der z-Ebene ermöglicht.

Das dritte Chassis kann bevorzugt mit einem Zahnrad und Antriebsritzel als Rotationsmotor versehen sein. Es versteht sich, dass die Rotation des dritten Chassis auch durch andere mechanische, elektrische, manuelle, pneumatische oder hydraulische Antriebsmittel hervorgerufen werden kann.

Es ist insbesondere von Vorteil, wenn das Reaktorgefäß unmittelbar an ein erstes Chassis für die horizontale Rotation um die x-Achse gekoppelt ist. Das erste Chassis ist unmittelbar an ein zweites Chassis für die vertikale Rotation gekoppelt, wobei das zweite Chassis wiederum unmittelbar an ein drittes Chassis für die Rotation in der z-Ebene gekoppelt ist. Diese Chassis-Anordnung ist insbesondere dann bevorzugt, wenn das Reaktorgefäß gerade horizontal ausgerichtet ist.

Vorzugsweise ist das Reaktorgefäß von einem zylinderförmigen oder einem rechteckigen Gefäß gebildet. Es kann auch jede andere quaderförmige, kubische, prismatische oder sonstige geeignete Form verwendet werden.

In einer ersten bevorzugten Ausführungsform ist die Materialträgereinheit im Reaktionsgefäß fixiert und bevorzugt mittig angeordnet. Die Materialträgereinheit ist dabei vorteilhafterweise über feste Verbindungen wie z.B. Kunststoff-, Glas-, oder Metallstege mit dem Reaktorgefäß gekoppelt.

Vorzugsweise ist das Reaktorgefäß mit einem oder mehreren Anschlüssen versehen, über welche Flüssigkeiten und/oder Gase durch den Reaktor hindurchleitbar sind. Solche Flüssigkeiten können ein Reaktionsmedium, insbesondere ein Nährmedium und/oder Waschlösungen, insbesondere Reinigungs- und Spülmedien enthalten.

Bei kontinuierlichen Verfahren ist eine Reinigung des Reaktionsgefäßes in bestimmten Zeitintervallen notwendig, da es z.B. häufig zu Ablagerungen an den Reaktorgefäßwänden oder Kontaminationen kommt, die die Produktqualität beeinträchtigen. Üblicherweise musste bisher hierfür das Verfahren unterbrochen werden und das Reaktorgefäß geöffnet werden.

Mit dem erfindungsgemäßen Reaktorsystem ist es nunmehr möglich, die Reinigung des Reaktorgefäßes ohne Unterbrechung des Verfahrens durchzuführen. Hierfür wird das Reaktorgefäß in eine Position gedreht, in der die Materialträgereinheit planar bzw. parallel zu einer der drei (x, y, z) - Achsen ausgerichtet ist. Beispielsweise wird das Reaktorgefäß in eine Position gedreht, in der die Materialträgereinheit planar bzw. parallel zur y-Achse ausgerichtet ist.

Im Folgenden wird die Reaktorgefäßwand unterhalb der Materialträgereinheit in einem ersten Schritt gereinigt. Dazu wird der flüssige Inhalt des Reaktionsgefäßes, z.B. das Reaktions-oder Nährmedium, bis zur vollständigen Entleerung des Reaktionsgefäßes abgeführt. Anschließend wird eine Reinigungslösung, wie z.B. ein alkalisches Medium, in den unterhalb der Materialträgereinheit befindlichen Abschnitt des Reaktionsgefäßes eingeleitet. Es wird jedoch nur soviel Reinigungslösung eingeführt, so dass die Materialträgereinheit nicht benetzt wird. Bevorzugt wird nur eine solche Menge an Reinigungslösung eingeführt, die eine Benetzung der Reaktorgefäßwand ermöglicht.

Anschließend wird das Reaktorgefäß nunmehr so gedreht, dass sich eine weitere Reaktorwand unterhalb der Materialträgereinheit befindet und die Materialträgereinheit parallel zur x-Achse ausgerichtet ist. Während des Reinigungsschrittes rotiert das Reaktionsgefäß z.B. horizontal um die x-Achse und sorgt somit für eine gleichmäßige Reinigung der Reaktorinnenwand.

Nach Beendigung des ersten Reinigungsschrittes wird das Reaktionsgefäß nunmehr so gedreht, dass die Materialträgereinheit planar, bzw. parallel zu einer weiteren Achse, z.B. zur y-Achse ausgerichtet ist, so dass der jetzt unter der Materialträgereinheit liegende Abschnitt gereinigt werden kann. Es versteht sich, dass auch eine Drehachse um die horizontale z-Achse geeignet ist, und die aus der Schwerkraft resultierende Bewegung der Materialträgereinheit nicht ausschließlich in vertikaler Richtung erfolgen muss.

Aufgrund der Drehbarkeit des Reaktorsystems ist es nunmehr möglich das Reaktorgefäß, insbesondere die Wände des Reaktorgefäßes, in einem geschlossenen System zu reinigen, ohne das das Reaktorgefäß oder die Materialträgereinheit geöffnet und berührt werden müssen.

Das erfindungsgemäße Reaktorsystem bietet somit mehrere Vorteile:
1. Vermeidung von körperlichem Kontakt zu z.B. sterilen, heißen, kalten, radioaktiven, explosiven, infektiösen, toxischen oder anderen gesundheitsschädlichen oder gefährlichen Ereignissen, Substanzen und Lebensformen.
2. Selbstreinigung gewährleistet gleich bleibend hohe Produktqualität, da die während des Produktionsprozesses entstehenden störenden Zwischenprodukte automatisch entfernt werden.
3. Selbstreinigung gewährleistet ununterbrochenen Produktionsprozess, da die Anreicherung von störenden lebenden oder toten Materialien verhindert wird.
4. Dämpfe und andere Stoffe, wie z.B. Lacke können unter kontrollierten Bedingungen hintereinander in ein und demselben System auf einer Matrix aufgebracht werden.

In einer weiteren bevorzugten Ausführungsform ist die Materialträgereinheit drei dimensional, insbesondere vertikal im Reaktorgefäß beweglich. Die Materialträgereinheit ist in dieser weiteren Ausführungsform mit Vorteil im Reaktorgefäß von außen, d.h. von außerhalb des Reaktionsgefäßes, lösbar arretierbar. Mit Vorteil ist die Materialträgereinheit kontaktlos magnetisch arretierbar. Die Magnetarretierung erfolgt bevorzugt durch Verwendung eines schaltbaren Elektromagneten.

Die Arretierung der Materialträgereinheit ist beliebig möglich, so dass die Materialträgereinheit in mindestens zwei unterschiedlichen Positionen im Reaktorgefäß arretierbar ist. So kann die Materialträgereinheit nach Drehung des Reaktorgefäßes um z.B. 180° um die horizontale x- Achse oder die horizontale z-Achse, im jeweils oberhalb der horizontalen Achse gelegenen Abschnitt des Reaktorgefäßes arretiert werden. Die Materialträgereinheit ist also z.B. im jeweils oberen Teil des Bioreaktors arretierbar.

Eine Drehbewegung des Reaktorgefäßes um die horizontale x-Achse des Reaktionsgefäßes führt zu einer langsamen und stetigen Vertikalbewegung der Materialträgereinheit durch das Lumen des Reaktorgefäßes. Dabei befindet sich die Materialträgereinheit unmittelbar nach der Drehbewegung im oberen Bereich des Reaktorgefäßes. Anschließend sinkt sie durch die Schwerkraft in den unteren Bereich des Reaktorgefäßes. Es versteht sich, dass auch eine Drehachse um die horizontale z-Achse geeignet ist, und die aus der Schwerkraft resultierende Bewegung der Materialträgereinheit nicht ausschließlich in vertikaler Richtung erfolgen muss.

Bei der vertikalen Bewegung der Materialträgereinheit infolge der Drehbewegung des Reaktorgefäßes wird das Reaktionsmedium wie z.B. Nährmedium durchmischt. Auf diese Weise wird das Material, wie z.B. Organismen optimal mit Nährstoffen und Sauerstoff versorgt, die beispielsweise aus einem Schlauchleitungssystem in das Reaktorgefäß gelangen. Die Drehbewegung des Reaktorgefäßes kann in regelmäßigen Intervallen erfolgen.

Insbesondere kann der jeweils unterhalb der arretierten Materialträgereinheit befindliche Teil des Reaktorgefäßes mit Anschlüssen versehen sein, über welche das Reaktorgefäß mit einer Quelle für Gase und/oder Reinigungs- und Pufferlösung verbindbar ist. Es können ferner Anschlüsse vorgesehen sein, über die weitere Substanzen, wie z.B. Zellen zugeführt und entnommen werden können. Ein Reinigungsmittel, wie z.B. eine alkalische Lösung als sterilisierendes Agens wird über den Anschluss in das Reaktorgefäß unterhalb der Materialträgereinheit eingelassen. Dabei werden dieser Teil und sämtliche Anschlüsse und Schläuche gereinigt. Anschließend wird das Reinigungsmittel abgelassen und ein Spülmittel, z.B. ein neutralisierendes Agens eingelassen. Schließlich kann das Reaktionsgefäß wieder mit dem Reaktionsmedium, z.B. einer Nährlösung und dergleichen für eine neue Kultivierungsphase befüllt werden. Der Reinigungsvorgang wird analog zu dem eben beschriebenen Verfahren für die bislang ungereinigte Hälfte des Reaktorgefäßes, insbesondere nach Rotation des Reaktionsgefäßes um 180° durchgeführt.

Die so verwirklichte Selbstreinigung während der Produktionsphase ermöglicht es, dass das Material, wie z.B. adhärente Zellen örtlich gebunden und quasi dauerhaft auch über lange Zeiträume bearbeitet werden können. Die Gefahr von Verschmutzung oder Kontaminationen wird wesentlich reduziert. Überflüssiges Substrat wird regelmäßig aus den Schläuchen und der inneren Oberfläche des Reaktorgefäßes entfernt.

Das Reaktorgefäß kann in einer weiteren Ausführungsform auch in seinem mittleren Bereich an einer Achse drehbeweglich aufgehängt sein. Dadurch lässt sich die Drehbewegung des Reaktorgefäßes leicht verwirklichen. Das Reaktorgefäß kann in beiden Drehrichtungen bewegt werden. Durch eine zweifache Bewegung um 180° gelangt das Reaktorgefäß zurück in seine Ausgangslage.

In einer besonders bevorzugten Ausgestaltung der Erfindung sind Mittel zum Auslösen einer Drehbewegung des Reaktorgefäßes um 180° um die horizontale x- oder z-Achse in auswählbaren Intervallen vorgesehen. Dann kann die Drehbewegung automatisch, beispielsweise durch einen Motor, eine Feder oder dergleichen ausgelöst werden. Ein Schrittmotor erhält hierfür über eine SPS Programmierung die erforderlichen Informationen für die Rotation.

Die vorliegende Erfindung basiert auf dem Gedanken, dass das Reaktorsystem z.B. das Wachstum adhärenter Zellen dadurch gewährleistet, dass sich die Apparatur im Kulturbetrieb selbst reinigt (Autosterilisation). Außerhalb der Materialträgereinheit, auf der im Falle einer Zellkultivierung die Zellen kultiviert werden, akkumulieren erfindungsgemäß in keinem Teil der Apparatur verdünnungsresistente Varianten und Substrate. Die Steuerung von Flüssigkeits- und Gasströmen erfolgt automatisch, d.h. über eine geeignete Ventilsteuerung mit rechnergesteuerten Ventilen.

Wenn Flüssigkeiten, wie z.B. Nährmedien und Waschlösungen und eine kontinuierliche Versorgung mit sterilen Gasen, beispielsweise Luft, zugeführt werden, dann arbeitet die Apparatur über eine quasi unbegrenzte Zeitdauer autonom. Der Betrieb der Anlage ist im Chemostat oder im Turbidostat Kulturregime anwendbar. Bei Bedarf können bestimmte Bestandteile von Zellen durch die Wirkung geeigneter Lösungen und Methoden abgetrennt und isoliert werden. Die Kulturbedingungen, insbesondere chemische und physikalische Parameter sind veränderbar.

Das Prinzip dieser Autosterilisation bietet neue Perspektiven hinsichtlich der Dauer und der Sterilität des Kulturbetriebs. Hohe Durchsatzraten, verbunden mit der Möglichkeit sich über einen langen Zeitraum an simulierte Prozess- oder Umweltbedingungen anzupassen, erhöhen die Wahrscheinlichkeit wirtschaftlich verwertbare Mikroorganismen zu generieren. Diese neu erworbenen Eigenschaften der Mikroorganismen sind aufgrund der langen Kultivierungszeit stabil in das Erbgut integriert und auch unter Prozessbedingungen abrufbar. Prozessbedingungen werden durch die Verwendung originaler Nährmedien, bzw. chemischer und physikalischer Parameter übernommen und in der Apparatur simuliert.

Das erfindungsgemäße Reaktorsystem ist somit insbesondere für die Kultivierung von biologischen Material, insbesondere von Enzymen, Zellen, Biofilmen oder Geweben einsetzbar und kann somit zur Fermentation, Biokatalyse, Optimierung von mikrobiellem Abbaupotential, Aufbau von organoiden Säugerzellstrukturen (tissue engineering), oder in Toxizitäts- und Bioverfügbarkeitsassays verwendet werden.

### Screening nach neuen Organismen und Enzymen

Durch Verwendung des erfindungsgemäßen Reaktorsystems können besondere Eigenschaften, wie beispielsweise die Säure- und Temperaturtoleranz von Mikroorganismen gezielt gefördert werden. Die aus den Kulturen gewonnenen neuen Biokatalysatoren (Enzyme) setzen Substrate hochspezifisch in die gewünschten Produkte um. Sie arbeiten im Vergleich zur chemischen Synthese industrieller Verfahren unter sehr milden Reaktionsbedingungen. Die entwickelten Biokatalysatoren ersetzen herkömmliche, oft teure, langwierige und umweltschädliche Verfahren.

Kaltwasserwaschprozesse sind energie- und ressourcenschonend. Dies erfordert bakterielle Enzyme, die in kaltem Wasser wirksam sind. Bisher unbekannte Mikroorganismen werden aus kaltem aquatischem Umweltmilieu isoliert und mit Hilfe des Bioreaktorssystems mit drehbaren Reaktorgefäßen über lange Zeiträume bei ca. 6-8°C kultiviert. Es resultieren kultivierbare Bakterienvarianten, deren Enzymsysteme an die gewählten Temperaturen optimal angepasst sind. Die Proteasen der Enzymsysteme können ihre Reinigungswirkung in einem Waschmittel voll entfalten.

Die gewünschten Eigenschaften der Mikroorganismen können gemäß einer Variation dieses Ausführungsbeispiels auch durch die Zugabe von DNA in das Reaktorsystem generiert werden.

### Optimieren molekularbiologisch veränderter Organismen

Es gibt bakterielle Varianten, deren Enzymsystem bei 6-8°C arbeitet. Die Varianten sind als Lieferant für Proteasen im Kaltwasserwaschprozess geeignet. Aufgrund von genetischen Manipulationen ist das Bakterium schlecht kultivierbar. Ein Einsatz im Produktionsprozess wäre dann nicht möglich.

Zur Behebung dieses Problems wird die genmanipulierte Variante unter geeigneten Bedingungen über lange Zeiträume kultiviert. Die Langzeitkultivierung ermöglicht weitere natürliche genetische Veränderungen (Mutationen) ausgehend von der genmanipulierten Variante. Während der Kultivierung setzen sich die Mutanten durch, die sich am schnellsten vermehren. Das Ende der Langzeitkultivierung ist dadurch charakterisiert, dass eine bakterielle Variante zur Verfügung steht, die die molekularbiologisch eingebrachte Veränderung trägt und sich nun zuverlässig kultivieren lässt.

### Verbesserte katalytische Aktivität unter Prozessbedingungen

Die Wirkung bisheriger Bakterienenzyme können durch Bleichmittel, Tenside, Enthärter und die geringe Alkalität des Wassers herabgesetzt werden. Dann können durch Dauerkultivierung Enzyme entwickelt werden, die an die jeweiligen Bedingungen angepasst werden. Dadurch werden die Produktionskosten reduziert und die Reinigungsleistung gesteigert. Der Bakterienstamm wird zur Anpassung an die Bedingungen gezielt mit den oben aufgeführten Problemstoffen und mit der gewünschten Alkalität über lange Zeiträume kultiviert. Es setzen sich bakterielle Varianten durch, deren Enzymsysteme optimal an die gewählten Bedingungen angepasst sind und die eine höhere Effizienz besitzen.

### Screening antibakterieller Materialeigenschaften

Mit der Dauerkultivierung können antibiotische Materialeigenschaften von konservierenden Beschichtungen und Oberflächen getestet werden. Solche konservierenden Beschichtungen und Materialoberflächen sind beispielsweise Lacke, Farben, Anti-Fouling Anstriche, Metall-Legierungen und Metalloberflächen. Sie werden für Materialien eingesetzt, die während ihrer Nutzung mikrobieller Besiedlung ausgesetzt sind. Für die Marketingstrategie und für die Qualitätssicherung können verbindliche und messbare Aussagen über die antibiotischen Materialeigenschaften gemacht werden. Hierzu werden die Materialien die während ihrer Nutzung mikrobieller Besiedlung ausgesetzt sind als Substrat in der Langzeitkultivierung mit den relevanten Mikroorganismen eingesetzt. Diese Mikroorganismen sind beispielsweise Bakterien, Algen oder Pilze. Aus der Langzeitkultivierung ergeben sich Erkenntnisse zur Haltbarkeit der Materialkonservierung gegen den mikrobiellen Bewuchs und zur Wirkung des Materials auf ausgewählte Organismen oder Organismengruppen.

### Biotestsystem für gesundheitsgefährdende Stoffe

Neue chemische Verbindungen, wie Kunststoffe und Polymere, können durch Dauerkultivierung auf mögliche Risiken überprüft werden. Hierfür wird die Biokompatibilität untersucht. Das Polymer wird zusammen mit Mikroorganismen über lange Zeiträume kultiviert. Die Biokompatibilität des geprüften Materials (Toxizität) kann nach Ablauf eines zuvor festgelegten Zeitraums beurteilt werden. Befindet sich ein Polymer bereits auf dem Markt, ohne biokompatibel zu sein, kann das Abbaupotential von Mikroorganismen so verändert werden, dass die Degradation des Polymers möglich wird. Dies wurde bereits anhand der Enzymoptimierung beschrieben.

### Herstellung gewebeähnlicher Strukturen (Tissue Engineering)

Eine Vielzahl von Zellteilung zur Entwicklung drei dimensionaler Gewebestrukturen ist nur durch lange Kultivierungszeiten zu erreichen. Hierbei kann die Zellträgereinheit so gestaltet sein, dass sie zugleich die Form für das aufzubauende Organ darstellt. Dementsprechend wird das Reaktorgefäß anwendungsbezogen angepasst. Die Zugabe von z.B. Chemikalien, wie z.B. Wachstumsfaktoren und Trägermaterialien, bzw. die sterile Entnahme von Zellen sind möglich.

### Alternative für Tierversuche

Langzeitkulturen ermöglichen eine Vielzahl von Zellteilungen und damit den Aufbau großvolumiger organoider Strukturen. Ergänzend zu Tierversuchen an Säugetieren können im Vorfeld neue Wirkstoffe an gewebeähnlichen Strukturen getestet werden. Es werden Zelllinien ausgewählt, die auch später das Zielorgan bilden, an dem der neue Wirkstoff ansetzt. Erste Versuchsreihen können außerhalb des tierischen Organismus annähernd in-vivo durchgeführt werden. Es ist ausreichend Material zur kontinuierlichen Probenentnahme vorhanden.

Des Weiteren ist jedoch auch ein Einsatz des vorliegenden Reaktorsystems zur Fertigung von Produkten aus der Halbleiter-, Optik- und Lasertechnologie, Bauteilen der Luft- und Raumfahrtechnik, der keimfreien Produktion von Lebensmitteln und Arzneimitteln und der Nanotechnologie vorstellbar, da das Reaktorsystem eine Produktion unter kontrollierten Bedingungen ermöglicht.

So wird das Reaktorsystem bevorzugt zur Abscheidung von Atomlagen auf dem Gebiet der Nanotechnologie verwendet.

Auch kann das erfindungsgemäße Reaktorsystem im industriellen Anlagenbau Verwendung finden, wie z.B. zum Steuern, Öffnen, Sperren, Dosieren, Mischen und Verteilen von Stoff-und Materialströmen, welche problematische Eigenschaften betreffend Temperatur, Sterilität, Strahlungsaktivität, Toxizität u.a. aufweisen.

Vorzugsweise sind mehrere Reaktorgefäße parallelgeschaltet. Dabei kann das gleiche Schlauchsystem mit Nährlösung und Reinigungsmitteln für alle Reaktorgefäße verwendet werden. Dadurch wird der experimentelle Durchsatz erhöht und physikalische und chemische Parameter sind für jedes der verwendeten Reaktorgefäße unabhängig voneinander einstellbar. Die Materialträgereinheit zur Verwendung in einem erfindungsgemäßen Reaktorsystem umfasst mindestens einen Materialträger oder Matrix, insbesondere flächigen Materialträger dessen Oberfläche mit Lamellen versehen ist. Mit Vorteil ist der Materialträger ein Zellträger. Der Materialträger kann aus einem magnetisierbaren Metall bestehen. Als bevorzugte Materialien werden vernickelter und galvanisierter Chromstahl verwendet.

Dem Aufbau des Reaktionsgefäßes entsprechend, kann die Materialträgereinheit einen Durchmesser und eine Höhe von wenigen Millimetern bis zu Zentimetern und Metern, bevorzugt von 10 mm bis 10 m haben und eine sowohl quaderförmige, kubische, prismatische oder sonstige geeignete Form aufweisen.

In einer bevorzugten Ausführungsform umfasst die bewegliche Materialträgereinheit mindestens eine obere und mindestens eine untere Platte, wobei die Platten über mindestens drei Stege miteinander verbunden sind. Die Unterseite der oberen Platte und die Oberseite der unteren Seite weisen bevorzugt Vorsprünge oder Schienen auf, in welche der flächige Materialträger eingeschoben wird. Die Materialträgereinheit kann somit bis zu 20 Materialträger umfassen.

Wie bereits erwähnt, kann das vorliegende Reaktorsystem zur Herstellung von Produkten unter kontrollierten, sterilen und keimfreien Bedingungen, wie im Bereich der Biologie, Biotechnologie, Chemie, Lebensmittel und Getränkeherstellung, Pharmazie, Halbleitertechnologie, Optik, Lack- und Farbindustrie verwendet werden.

Insbesondere kann die Reaktorsystem zur Veränderung von biologischem Material, zur Kultivierung von Zellen, Biofilmen und Geweben für die Enzymoptimierung, für in-Vitro-Tests und für die Herstellung gewebeähnlicher Strukturen (Tissue-Engineering) eingesetzt werden.

Die Herstellung von Produkten in einem Reaktorsystem mit einem um mindestens eine der drei (x, y, z) Achsen drehbaren Reaktorgefäß in dem eine Materialträgereinheit angeordnet ist, insbesondere unter Verwendung des erfindungsgemäßen Reaktorsystems, kann nach einem bevorzugt kontinuierlich durchgeführten Verfahren erfolgen, das gekennzeichnet ist durch die folgenden Schritte:
(a) Abscheiden oder Auftragen einer Zusammensetzung auf der Materialträgereinheit;
(b) ggf. Arretieren der Materialträgereinheit im jeweils oberhalb der horizontalen Achse gelegenen Abschnitt des Reaktorgefäßes;
(c) Drehen des Reaktorgefäßes;
(d) Reinigen des Bereichs in dem Reaktorgefäßes unterhalb der Materialträgereinheit; und
(e) ggf. Lösen der Arretierung der Materialträgereinheit, so dass diese sich in den gereinigten Bereich in dem Reaktionsgefäß bewegt.

So wird in einer bevorzugten Ausführungsform im Bereich der Nanotechnologie ein zu beschichtendes Substrat mit der Materialträgereinheit in das Reaktorgefäß eingebracht. Anschließend wird das abzuscheidende Material mit einem geeigneten Trägergas unter den erforderlichen Druck- und Temperaturbedingungen in das Reaktorgefäß eingetragen, wo sich dieses auf dem Substrat ablagert. Anschließend erfolgt die Reinigung des Reaktorgefäßes durch Spülen mit geeigneten Gasen wie z.B. Edelgase oder Ammoniak, um das Reaktorgefäß auf den nächsten Zyklus vorzubereiten.

In einer weiteren bevorzugten Ausführungsform des Verfahrens erfolgt die Kultivierung von Zellen, Biofilmen und Geweben in einem Reaktorsystem mit einem um mindestens eine horizontale Achse drehbaren Reaktorgefäß in dem eine Materialträgereinheit angeordnet ist, mit den folgenden Schritten:
(a) Kultivieren der Kultur auf der Materialträgereinheit;
(b) ggf. Arretieren der Materialträgereinheit im jeweils oberhalb der horizontalen Achse gelegenen Abschnitt des Reaktorgefäßes;
(c) Drehen des Reaktorgefäßes;
(d) Reinigen des Bereichs in dem Reaktorgefäß unterhalb der Materialträgereinheit; und
(e) ggf. Lösen der Arretierung der Materialträgereinheit, so dass diese sich in den gereinigten Bereich in dem Reaktionsgefäß bewegt.

Dabei werden die Schritte (a) bis (e) vorzugsweise in regelmäßigen Zeitabständen, beispielsweise nach etwa 70 bis 74 Stunden, wiederholt.

Die Vorteile des erfindungsgemäßen Reaktorsystems insbesondere bei der Kultivierung von biologischem Material im Vergleich zu herkömmlichen Lösungen sind die in der Autosterilisation (Selbstreinigung der Anlage) begründeten, zeitlich quasi unbegrenzten Kultivierungszeiten. Das System bietet ferner die Möglichkeit zeitgleich mehrere Parallelansätze durchführen zu können. Dadurch ergibt sich eine Erhöhung des Durchsatzes. Physikalische und chemische Parameter, wie die Temperatur, der pH-Wert oder die Substratkonzentrationen können im System verändert werden. Die Parameter können auch gezielt eingesetzt werden um wirtschaftlich nutzbare mikrobielle Varianten und ihre Produkte, wie Enzyme, zu generieren.

In dem Bioreaktorsystem ist die Entnahme und Befüllung des Reaktors mit organischen und anorganischen Substanzen möglich.

Die Erfindung wird nachfolgend unter Bezugnahme auf die Figuren an mehreren Ausführungsbeispielen näher erläutert. Es zeigen:
- Fig. 1: eine schematische Darstellung einer bevorzugten Ausführungsform des Grundaufbaus des Reaktorsystems in Seitenansicht,
- Fig. 2A: eine schematische Darstellung der ersten bevorzugten Ausführungsform des Reaktorsystems mit einer fixierten Materialträgereinheit in Frontansicht, wobei sich die Materialträgereinheit in einer horizontalen Position befindet,
- Fig. 2B: eine schematische Darstellung der ersten bevorzugten Ausführungsform des Reaktorsystems mit einer fixierten Materialträgereinheit in Frontansicht, wobei sich die Materialträgereinheit in einer vertikalen Position befindet,
- Fig. 3A: eine schematische Darstellung einer weiteren Ausführungsform eines kubischen Reaktorsystems mit einer fixierten Materialträgereinheit in Frontansicht, wobei sich die Materialträgereinheit in einer parallel zur x-Achse vertikalen Position befindet;
- Fig. 3B: eine schematische Darstellung einer weiteren Ausführungsform eines kubischen Reaktorsystems mit einer fixierten Materialträgereinheit in Frontansicht, wobei sich die Materialträgereinheit in einer horizontalen Position befindet;
- Fig. 3C: eine schematische Darstellung einer weiteren Ausführungsform eines kubischen Reaktorsystems mit einer fixierten Materialträgereinheit in Frontansicht, wobei sich die Materialträgereinheit in einer parallel zur z-Achse vertikalen Position befindet;
- Fig. 4A: eine Anordnung aus Figur 2B in der Flüssigkeiten in den Bereich des Reaktionsgefäßes geleitet werden, der unterhalb der Materialträgereinheit liegt.
- Fig. 4B: eine Anordnung aus Figur 2B in der sich das Reaktionsgefäß im Uhrzeigersinn in eine horizontale Position dreht und Flüssigkeiten in den Bereich des Reaktionsgefäßes geleitet werden, der unterhalb der Materialträgereinheit und parallel zur horizontalen x-Achse liegt.
- Fig. 4C: eine Anordnung aus Figur 2B in der sich das Reaktionsgefäß in einer horizontale Position befindet und Flüssigkeiten in den Bereich des Reaktionsgefäßes geleitet werden, der unterhalb der Materialträgereinheit und parallel zur horizontalen x-Achse liegt,
- Fig. 5: eine schematische Darstellung einer weiteren bevorzugten Ausführungsform des Reaktorsystems mit einer vertikal beweglichen Materialträgereinheit,
- Fig. 6: eine schematische Darstellung einer Materialträgereinheit für ein drehbares Reaktorgefäß,
- Fig. 7: eine schematische Darstellung einer bevorzugten Ausführungsform des Grundaufbaus des Reaktorsystems in Seitenansicht und einer zugehörigen Magnetarretierung,
- Fig. 8: eine schematische Darstellung eines Bioreaktorsystems mit einem drehbaren Reaktorgefäß, in dem der Verlauf der Zuleitungen und die Lage der Ventile dargestellt ist,
- Fig. 9: eine Anordnung aus Figur 8, in der der Weg der Nährmittellösung illustriert ist,
- Fig. 10: eine Anordnung aus Figur 8, in der der Weg der in dem Reaktorgefäß enthaltenden Flüssigkeit zum Abfallsammler illustriert ist,
- Fig. 11: eine Anordnung aus Figur 8, in der der Weg der Natronlauge illustriert ist,
- Fig. 12: eine Anordnung aus Figur 8, in der der Weg der Pufferlösung illustriert ist,
- Fig. 13: eine Anordnung aus Figur 9, in der der Weg der Nährmittellösung bei gegenüber Figur 9 gedrehtem Reaktorgefäß illustriert ist,
- Fig. 14: eine Anordnung aus Figur 10, in der der Weg der in dem Reaktorgefäß enthaltenden Flüssigkeit zum Abfallsammler bei gegenüber Figur 10 gedrehtem Reaktorgefäß illustriert ist,
- Fig. 15: zeigt die Anordnung aus Figur 11, in der der Weg der Natronlauge bei gegenüber Figur 11 gedrehtem Reaktorgefäß illustriert ist,
- Fig. 16: eine Anordnung aus Figur 12, in der der Weg der Pufferlösung bei gegenüber Figur 12 gedrehtem Reaktorgefäß illustriert ist,
- Fig. 17: eine schematische Darstellung eines Bioreaktorsystems mit mehreren parallel geschalteten, drehbaren Reaktorgefäßen für einen erhöhten experimentellen Durchsatz und variierbare Kulturbedingungen,
- Fig. 18: eine schematische Darstellung einer Magnetarretierung für ein drehbares Reaktorgefäß in einem Bioreaktor,
- Fig. 19: eine perspektivische Darstellung der Magnetarretierung aus Figur 18.

### Ausführungsbeispiel 1: Reaktorsystem mit fixierter Materialträgereinheit

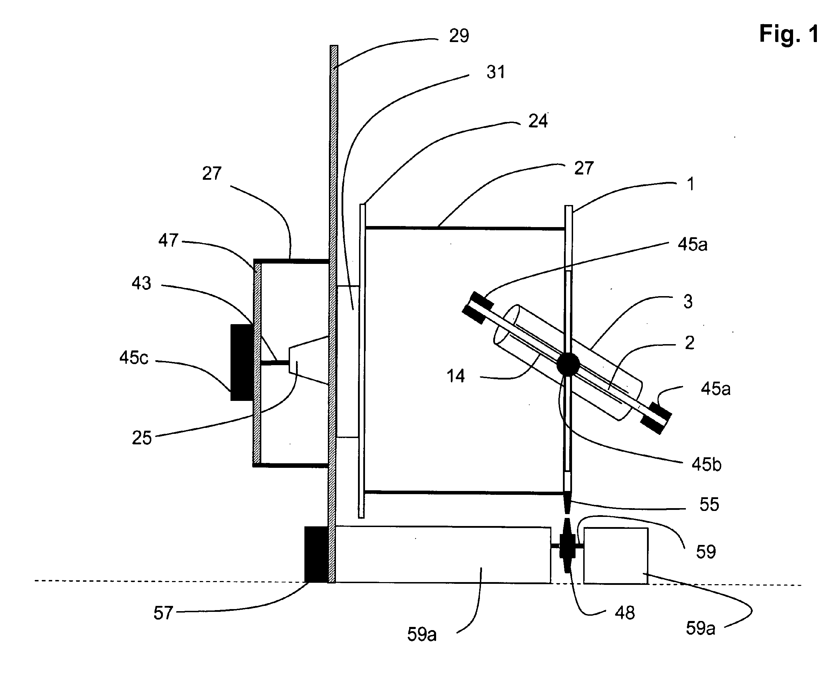

Figur 1 zeigt den schematischen Aufbau des Reaktorsystems. Das Reaktionsgefäß 3 weist das Chassis 2 zur vertikalen Rotation um die y-Achse auf. Diese Rotation um die y-Achse wird von zwei Schrittmotoren 45a hervorgerufen, die jeweils an der Achse außerhalb des Reaktorgefäßes 3 angebracht sind. Im Reaktorgefäß 3 befindet sich die Materialträgereinheit 14. Das Reaktorgefäß 3 ist über Chassis 2 des Weiteren an dem Chassis 1 für die horizontale Rotation um die x-Achse angebracht, welche durch den Schrittmotor 45b veranlasst wird. Die Kopplung des Chassis 1 an das Chassis 24 zur Bewegung in der z-Ebene erfolgt über die Distanzbolzen 27. Das Chassis 24 wiederum ist über ein Kugellager 31 an eine Montagewand 29 gekoppelt. Die Rotation des Chassis 24 wird durch den Schrittmotor 45c hervorgerufen, der über die Antriebswelle 43 und die Kupplung 25 mit dem Chassis 24 gekoppelt ist.

Die Antriebswelle 59 ist zur Kompensation der Last des Chassis 24 und dessen Bauteilen in den Antriebwellenlagern 59 a gelagert. Die Anordnung gewährleistet die reibungsarme und gleichmäßige Rotation des Antriebsritzels 48. Das Antriebsritzel 48 greift in das auf Chassis 1 befestigte Zahnrad 55 und lässt bei Betrieb von Schrittmotor 57 Chassis 1 in der horizontalen z-Ebene rotieren. Es versteht sich, dass zur Verteilung der Last von Chassis 1 dieses auch in ein Kugellager aufgenommen werden kann und die Rotation um die horizontale z-Achse ausschließlich über den Schrittmotor 45c erfolgen kann.

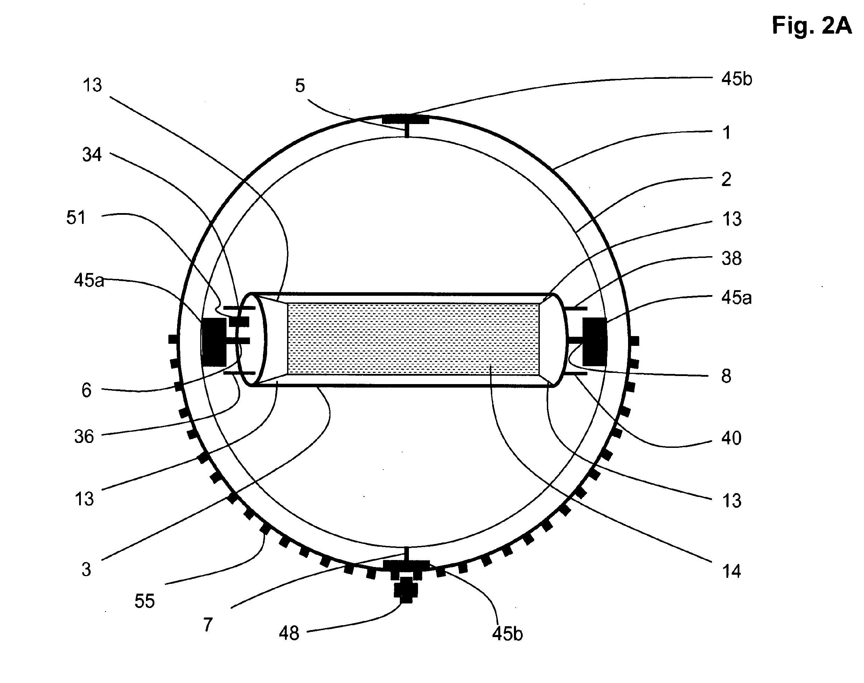

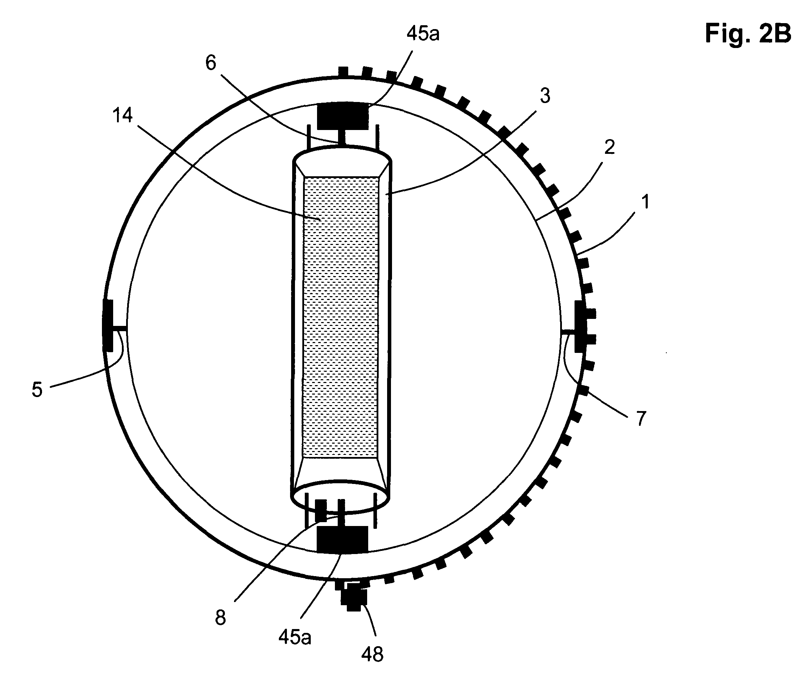

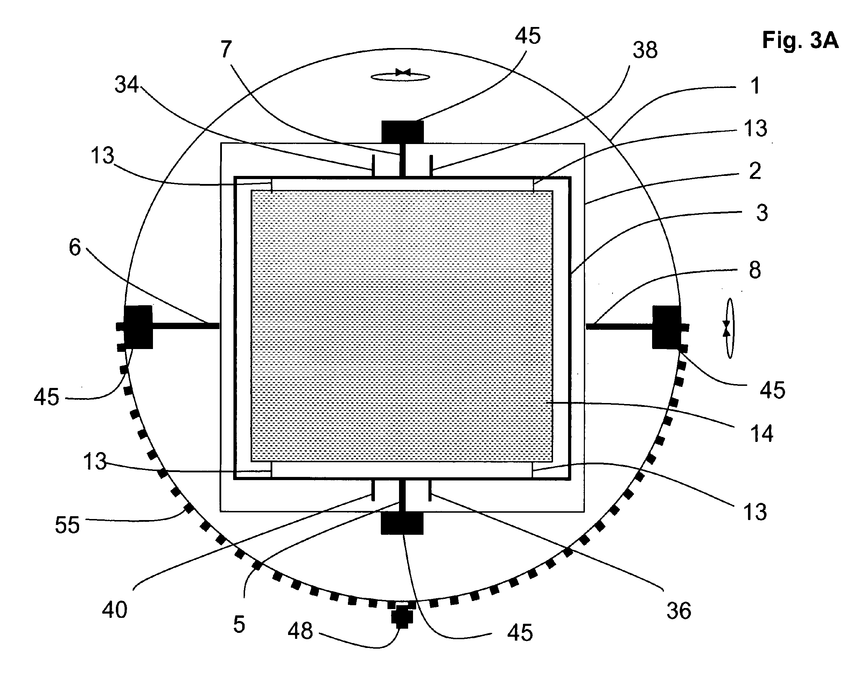

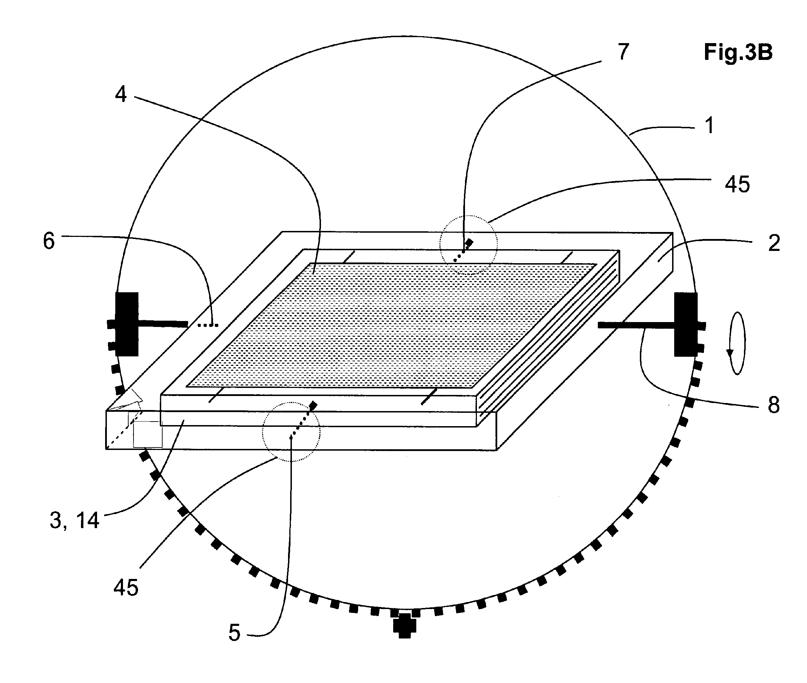

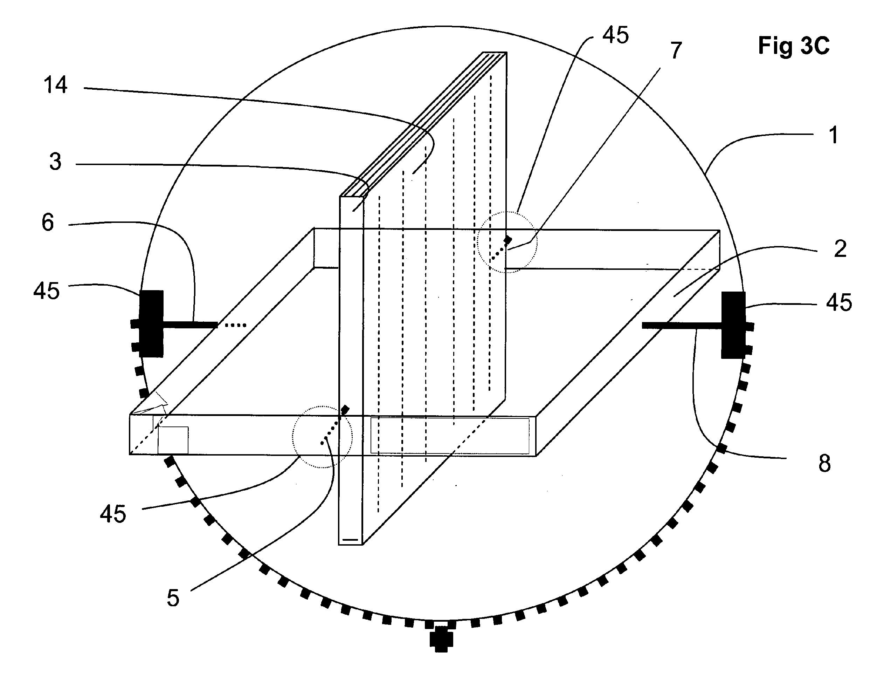

Figuren 2A-B und 3A-C zeigen eine erste Ausführungsform des erfindungsgemäßen Reaktorsystems mit einer fixierten Materialträgereinheit 14. In Figur 2A ist eine Frontansicht des Reaktorsystems in horizontaler Position und in Figur 2B eine Frontansicht des Reaktorsystems in vertikaler Position dargestellt, während in den Figuren 3A-C die möglichen horizontalen und vertikalen Drehungen einer rechteckigen Ausführung des Reaktorgefäßes 3 mit der fixierten Materialträgereinheit 14 zu sehen sind.

In Figur 2A ist das Reaktorgefäß 3 mit der darin fixierten Matrix oder Materialträgereinheit 14 an das Chassis 2 gekoppelt, welches eine Rotationsachse um die horizontale x-Achse 6, 8 aufweist. Das Chassis 2 ermöglicht somit eine Rotation des Reaktorgefäßes 3 um seine horizontale x-Achse 6, 8.

Das Chassis 2 ist an das Chassis 1 gekoppelt, welches eine Rotationsachse um die vertikale y-Achse 5, 7 aufweist. Das Chassis 1 ermöglicht somit eine Rotation des Reaktorgefäßes 3 um seine vertikale y-Achse 5, 7.

Wie in Fig 1 dargestellt, sind Chassis 1, 2 wiederum bevorzugt an das Chassis 24 gekoppelt, welches eine Rotation der Reaktorgefäßanordnung umfassend Reaktorgefäß 3 mit ersten und zweiten Chassis 1, 2 in der z-Ebene ermöglicht.

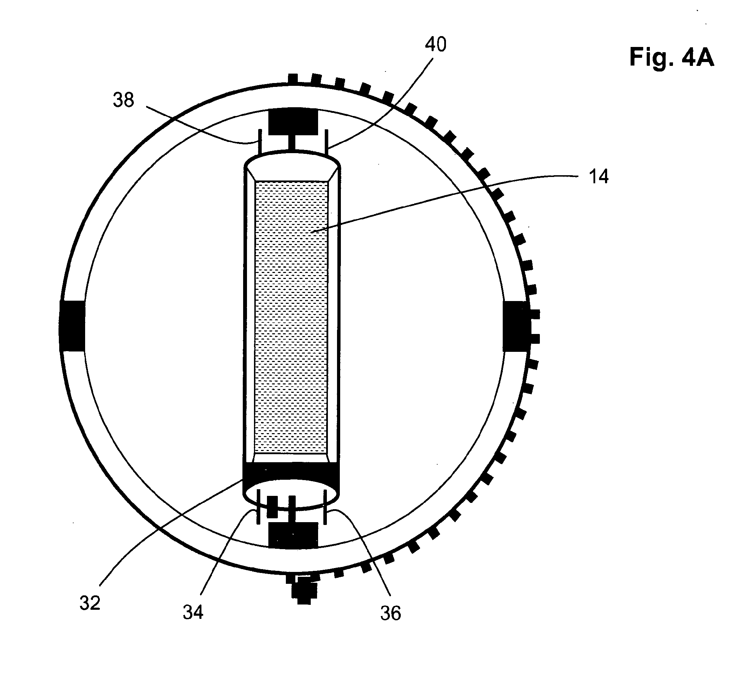

In Folge der Rotation des Chassis 1 mit Chassis 2 und Reaktorgefäß 3 um die horizontale z-Achse wird die Materialträgereinheit in eine vertikale Position gebracht (Fig 4A). Diese Position ermöglicht die Reinigung eines ersten Teilabschnittes des Reaktionsgefäßes 3. Nach dieser Drehung um die z-Achse ist die Materialträgereinheit 14 somit planar zu einer ersten der Reaktorgefäßwände ausgerichtet, wobei sich diese Reaktorgefäßwand unterhalb der Materialträgereinheit 14 befindet. Diese Reaktorgefäßwand wird in einem ersten Schritt gereinigt.

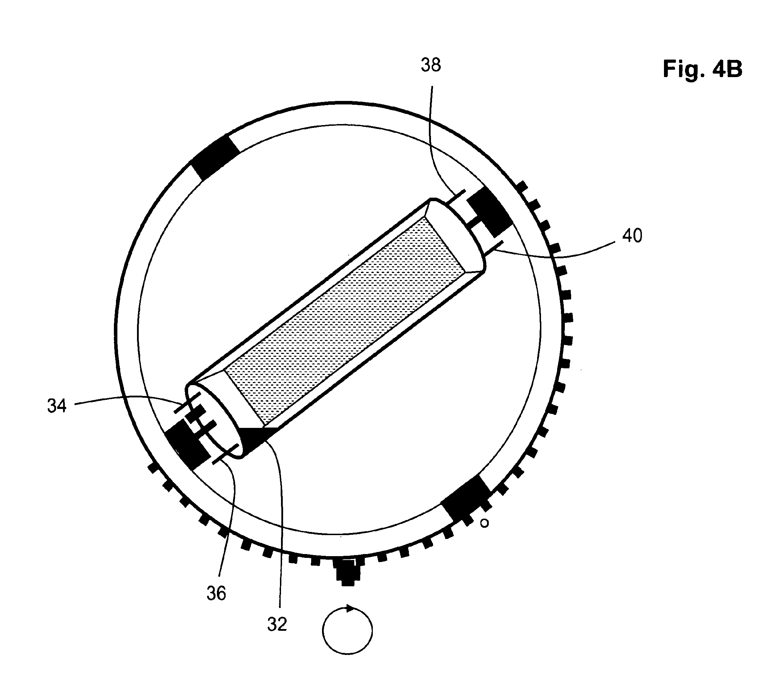

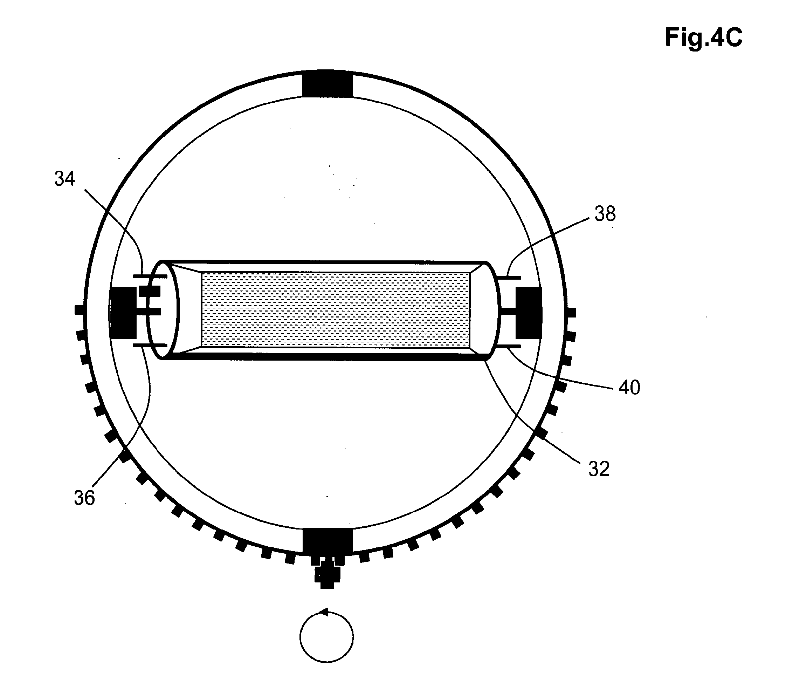

Hierfür wird das Reaktions- oder Nährmedium bis zur vollständigen Entleerung des Reaktionsgefäßes abgeführt. Anschließend wird ein alkalisches Medium zur Sterilisation in Form der Flüssigkeit 32, in den unterhalb der Materialträgereinheit 14 befindlichen Abschnitt des Reaktionsgefäßes 3 eingeleitet (Fig. 4A). Es wird jedoch nur soviel Reinigungslösung 32 eingeführt, dass die Materialträgereinheit nicht benetzt wird. Bevorzugt wird nur eine solche Menge an Reinigungslösung 32 eingeführt, die eine Benetzung der Reaktorgefäßwand ermöglicht. Das Reaktorgefäß wird nunmehr so um seine Rotationsachsen gedreht, dass sich jeweils eine weitere Reaktorwand unterhalb der Materialträgereinheit 4 befindet (Fig 4B, C). Die Materialträgereinheit 14 ist wiederum parallel zu der zu reinigenden Reaktorwand angeordnet. Das eingeleitete Volumen Reinigungslösung wird über alle inneren Reaktorwände geleitet, ohne die Materialträgereinheit zu benetzen. Die Reinigungslösung wird abgeführt und anschließend Pufferlösung zur Neutralisation eingeleitet. Der Ablauf der Neutralisation entspricht den eben beschriebenen Schritten der Sterilisation.

### Ausführungsbeispiel 2: Reaktorsystem mit vertikal beweglicher Materialträgereinheit

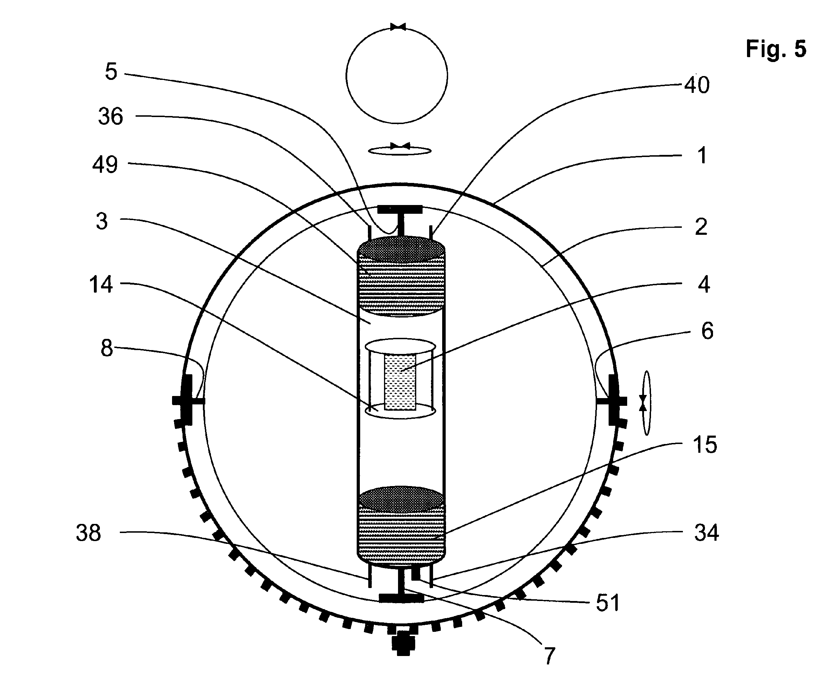

Figur 5 zeigt eine zweite Ausführungsform des erfindungsgemäßen Reaktorsystems.

In dieser Ausführungsform ist das Reaktorgefäß 3 zylindrisch ausgebildet und ist mit der sich darin befindenden Materialträgereinheit 14 an das Chassis 2 gekoppelt, welches eine Rotationsachse um die vertikale y-Achse aufweist. Chassis 2 ermöglicht somit die Rotation des Reaktorgefäßes 3 um seine vertikale y-Achse. Chassis 2 ist an Chassis 1 gekoppelt, welches eine Rotationsachse um die horizontale x-Achse aufweist. Chassis 1 ermöglicht daher die Rotation des Reaktorgefäßes 3 um seine horizontale x-Achse.

Chassis 2, 1 sind wiederum bevorzugt an ein drittes Chassis 24 gekoppelt, welches eine Rotation der Reaktorgefäßanordnung umfassend Reaktorgefäß 3 mit den Chassis 2, 1 in der z-Ebene ermöglicht.

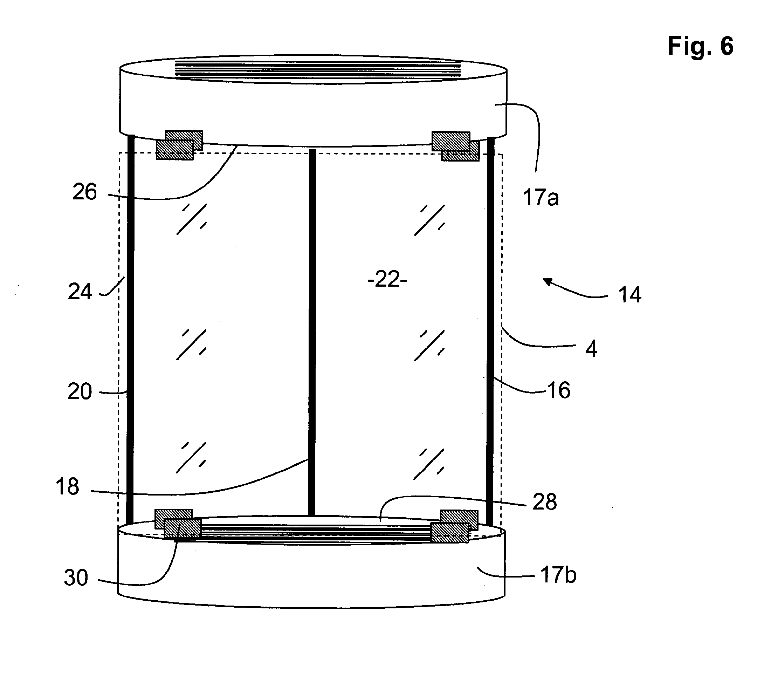

In Figur 6 ist eine allgemein mit 14 bezeichnete Zellträgereinheit dargestellt. Die Zellträgereinheit weist vorzugsweise eine obere und eine untere, runde Platte 17a und 17b auf. Die Platten 17a und 17b sind über drei Stege 16, 18 und 20 miteinander verbunden. Zwischen den Platten 17a und 17b ist ein Hohlraum 22 gebildet. In dem Hohlraum sind flächige Zellträger 4, zum Beispiel Objektträger mit Zellkulturen, einschiebbar. Zu diesem Zweck weist die Unterseite 26 der oberen Platte 17a und die Oberseite 28 der unteren Platte 17b längliche Vorsprünge oder Schienen 30 auf. Die Zellträger 4 werden zwischen den Vorsprüngen 30 eingeschoben und dort beabstandet zueinander gehalten. Die Zellträgereinheit 14 besteht aus magnetisierbarem, rostfreiem Edelstahl, der Laugen- und Säurebeständig ist. Sie ist ferner bis zumindest 121°C autoklavierbar und leicht.

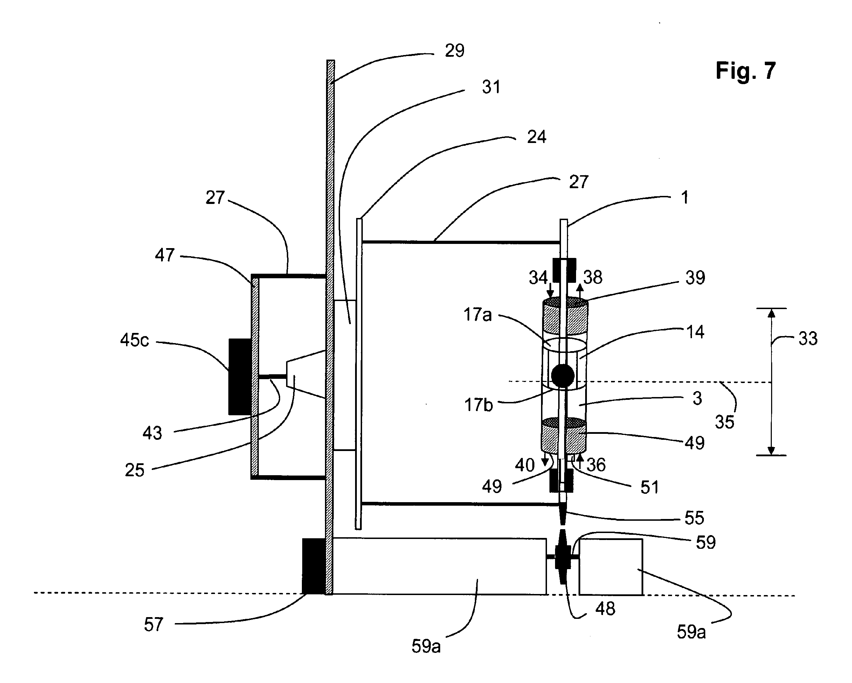

Die Zellträgereinheit 14 wird vorzugsweise in ein zylindrisches Reaktorgefäß 3 eingesteckt (Fig. 7). Das Reaktorgefäß 3 ist ein durchsichtiges Gefäß, beispielsweise aus Glas. Das Reaktorgefäß 3 hat einen geringfügig größeren Durchmesser als die Zellträgereinheit 14. Die Mantellänge 33 des Reaktorgefäßes 3 ist zumindest doppelt so groß, wie die Länge der Zellträgereinheit 14. Die Zellträgereinheit 14 ist dadurch in dem Reaktorgefäß 3 vertikal beweglich. Das Reaktorgefäß 3 ist bezüglich einer horizontalen Ebene 35 symmetrisch aufgebaut.

Das Reaktorgefäß 3 wird in Chassis 2 gehalten. Chassis 2 ist über Chassis 1 und Chassis 24 auf dem Kugellager 31 gelagert und wird über eine Kupplung durch den Schrittmotor 45c angetrieben. Der Elektromotor 45c sitzt an einem Chassis 47. Auf diese Weise wird das Reaktorgefäß 3 über Chassis 1,2 frei drehbar in Chassis 24 gehalten. Mit dem Elektromotor 45 kann das Reaktorgefäß 3 um die horizontale z-Achse gedreht werden.

Der Elektromotor 57 ist über Antriebswelle 59 und zwei Antriebswellenlager 59a mit dem Antriebsritzel 48 für das Zahnrad 55 von Chassis 1 verbunden. Auf diese Weise ist das Reaktorgefäß 3 über Chassis 1 über Antriebswelle 59 um die horizontale z-Achse frei drehbar, wenn aufgrund des hohen Gewichtes von Reaktorgefäß 3 der Antrieb über die Welle 45 nicht ausreicht. Im oberen und unteren Drittel des Reaktorsgefäßes 3 ist jeweils ein schaltbarer Elektromagnet 39, 49 befestigt.

Wenn das Reaktorgefäß 3 mit den Elektromotoren 45c, 57 gedreht wird, befindet sich die darin befindliche Zellträgereinheit 14 zunächst im oberen Bereich des Reaktorgefäßes 3, bevor es aufgrund der Schwerkraft absinkt. In diesem Moment kann die Zellträgereinheit 14 berührungslos in ihrer Lage arretiert werden, indem die Elektromagneten 39, bzw. 49 angeschaltet und eine Magnetkraft auf die Metallplatte 17a bzw. 17b der Zellträgereinheit 14 ausübt.

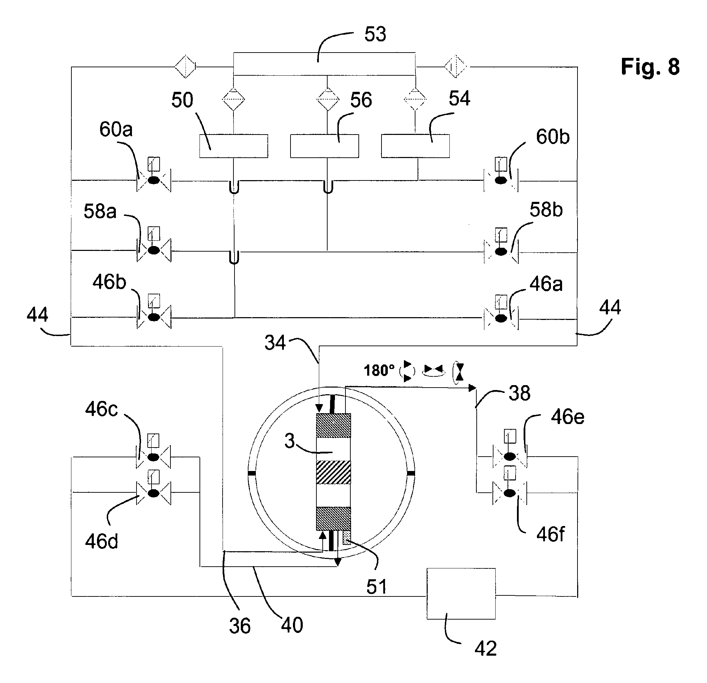

An der oberen und unteren Stirnseite des Reaktorgefäßes sind jeweils ein Einlass 34 bzw. 36 und ein Auslass 38 bzw. 40 vorgesehen (Fig. 7, 8). Der Auslass 38 bzw. 40 ist über einen Siphon mit einem Abfallsammler 42 verbunden. Für die Beschickung und Entnahme organischer und anorganischer Substanzen ist weiterhin der Ein- und Auslassanschluss 51 vorgesehen. Über ein nachstehend beschriebenes Ventilsystem können unterschiedliche Flüssigkeiten, wie Gase, Nähr- und Reinigungsflüssigkeiten, durch das Bioreaktorsystem geleitet werden.

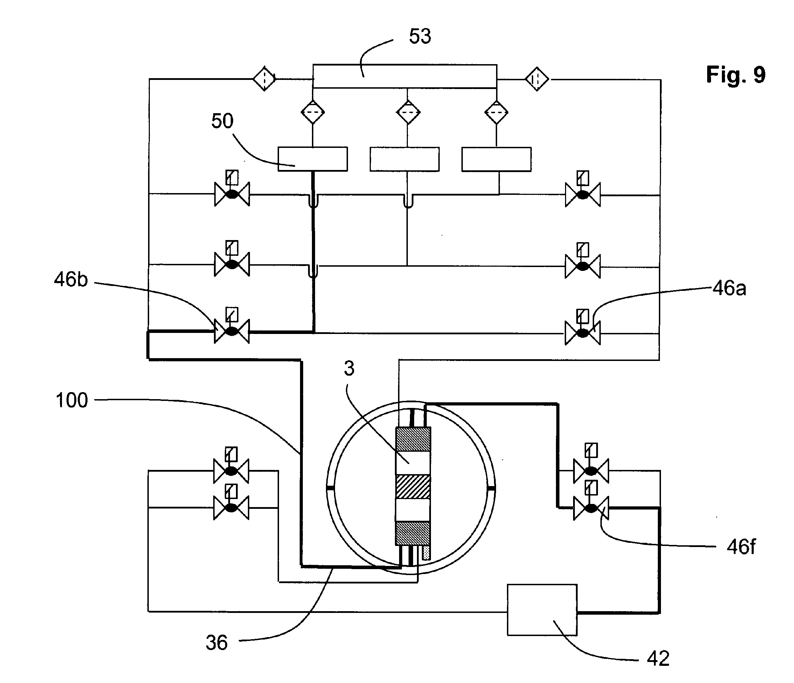

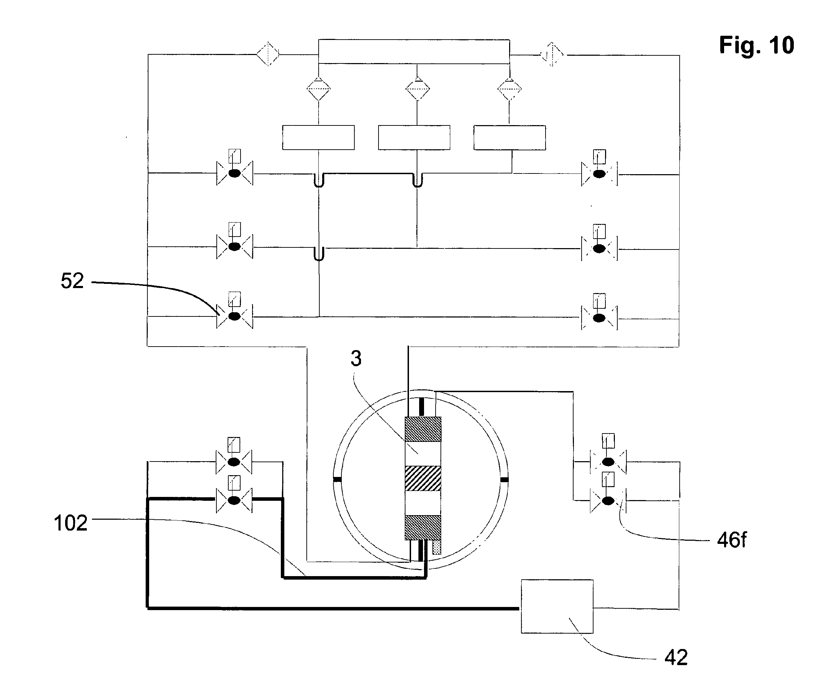

Die Einlassanschlüsse 34 bzw. 36 sind die gemeinsame Zuleitung für die Reservoire 50, 54 und 56. Ventile 46 a-f steuern den Zufluss für das Nährmedium. In die Zuleitungen 34 und 36 kann Reaktionsmedium, insbesondere Nährmedium aus einem Reservoir 50 eingelassen werden. Der Weg 100 des Nährmediums durch das Reaktorgefäß 3 ist in Figur 9 fett dargestellt. Dies wird über die Ventile 46a,b gesteuert. Weiterhin sind Reservoire für eine Gasquelle (z.B. Druckluft bei 0,6 bar) 53, Natronlauge 54 und Pufferlösung 56 vorgesehen. Diese werden über Ventile 58a, b und 60a,b in die Einlassanschlüsse 34 und 36 eingelassen. Der Weg 102 der in dem Reaktorgefäß 3 vorhandenen Flüssigkeit zum Entleeren des Reaktorgefäßes 3 in den Abfallsammler 42 ist in Figur 10 dargestellt.

Für die Dauerkultivierung von Mikroorganismen wird die Zellträgereinheit 14 mit den Kulturen in das Reaktorgefäß 3 eingesetzt. Eine geeignete Nährlösung wird über den unteren Einlass 36 eingelassen (Figur 9). In regelmäßigen Abständen von bspw. 5 Minuten wird das Reaktorgefäß 3 um 180° gedreht. Das Reaktorgefäß 3 ist zu diesem Zweck drehbar gelagert. Durch das Drehen wird die Nährlösung in ausreichendem Maße bewegt. Alle Mikroorganismen werden gleichmäßig mit Nährlösung und Sauerstoff versorgt.

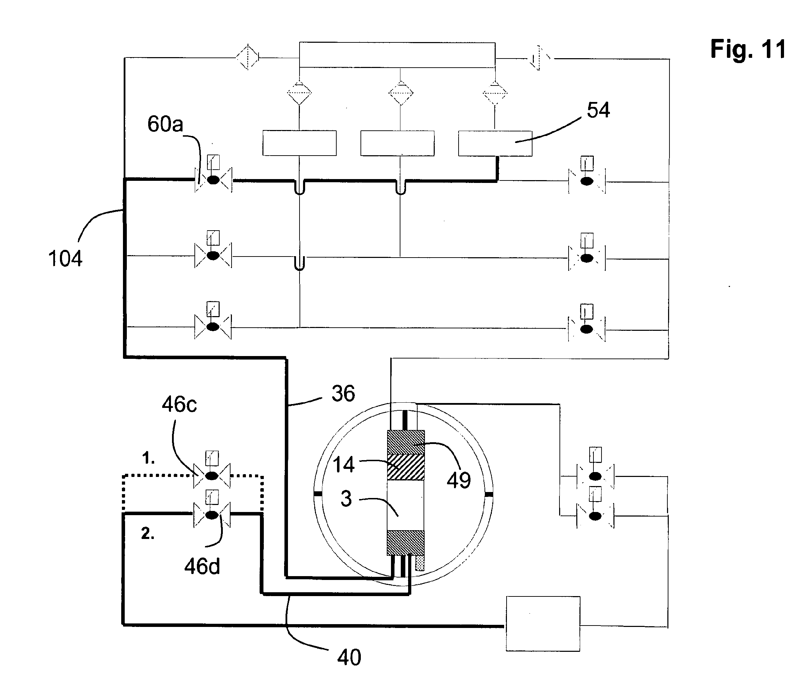

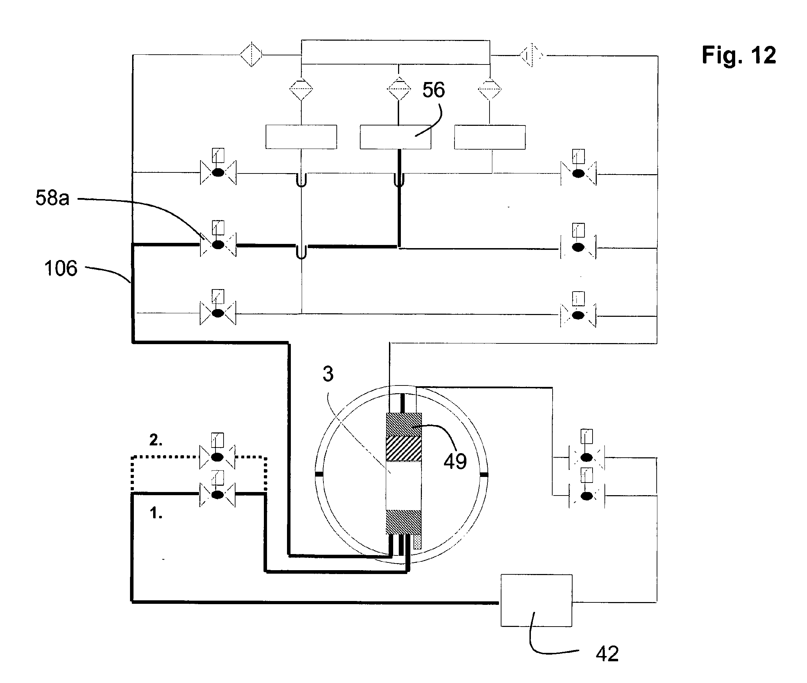

Für die Sterilisation des linken Anlagenabschnitts wird das Reaktorgefäß bspw. alle 72 h um 180° gedreht und die Zellträgereinheit 14 mittels Magnetarretierung 49 in der oberen Lage arretiert (Figur 11). Anschließend wird das Ventil 60 a geöffnet und Natronlauge in den Bereich unterhalb der Zellträgereinheit 14 eingelassen. Der Weg 104 der Natronlauge ist in Figur 11 fett dargestellt. Das Einfüllvolumen wird über die Siphonwirkung der Leitung 40 und durch Ventil 60 a so gesteuert, dass nur etwas mehr als das halbe Reaktorgefäß 3 im unteren Bereich mit dem sterilisierenden Agens befüllt werden kann. Das sterilisierende Agens reinigt neben den Wandungen des Reaktorgefäßes 3 auch die Zuleitung, den Einlass 36 und den Auslass 40. Anschließend wird das sterilisierende Agens durch Öffnen des Auslasses 40 erst über Ventil 46c und dann über Ventil 46d abgelassen. Zur Vermeidung von unerwünschten Veränderungen des pH-Wertes innerhalb des Reaktorgefäßes 3 wird anschließend auf gleiche Weise ein neutralisierendes Agens eingelassen. Der Weg 106 des neutralisierenden Agens in Form eine Pufferlösung ist in Figur 12 fett dargestellt. Für die Neutralisation wird das Ventil 58 für eine einstellbare Zeit geöffnet. Nach diesem Reinigungsvorgang sind der untere Teil des Reaktorgefäßes 3 und alle genutzten Zu- und Ableitungen frei von unkontrolliert wachsenden Zellen, Zellrückständen und Nährmediumsresten. Das Reaktorgefäß 3 kann wieder mit Nährmedium befüllt werden. Schließlich wird die Magnetarretierung 49 gelöst. Die Zellträgereinheit 14 sinkt in der Flüssigkeit nach unten. Eine neue Kultivierungsphase beginnt.

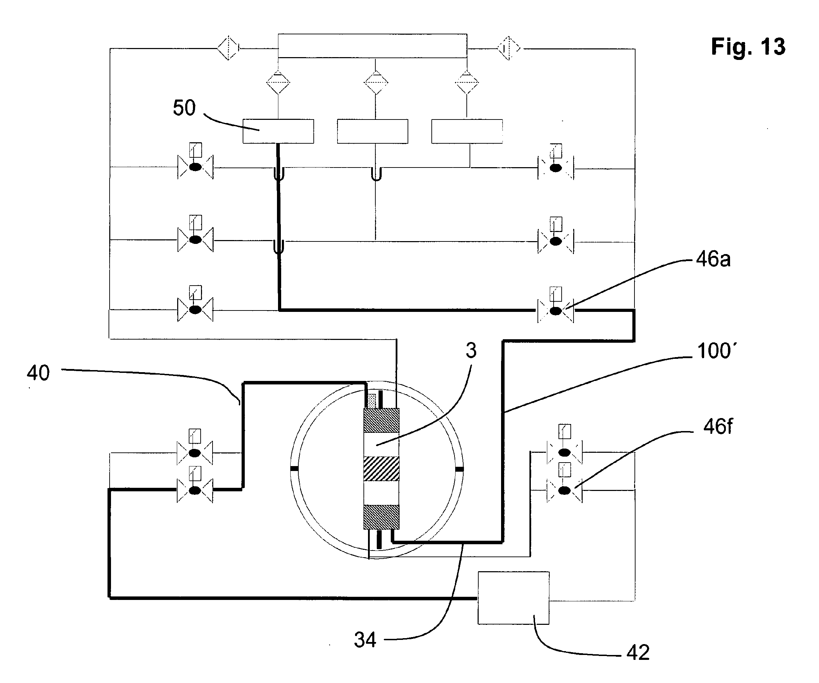

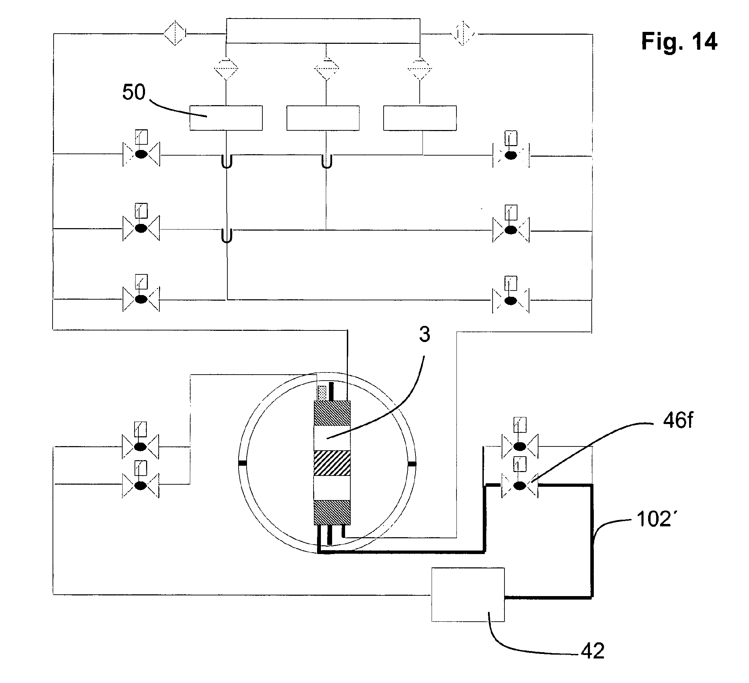

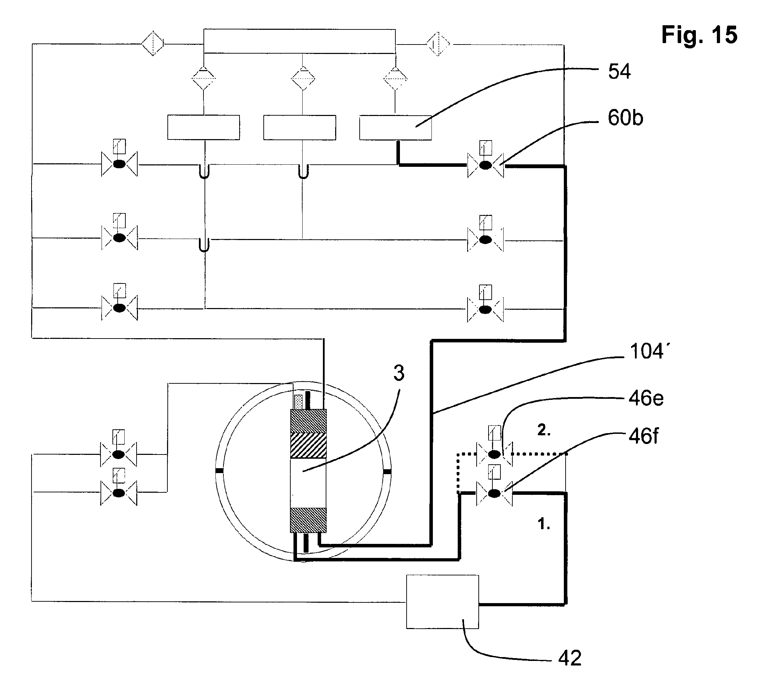

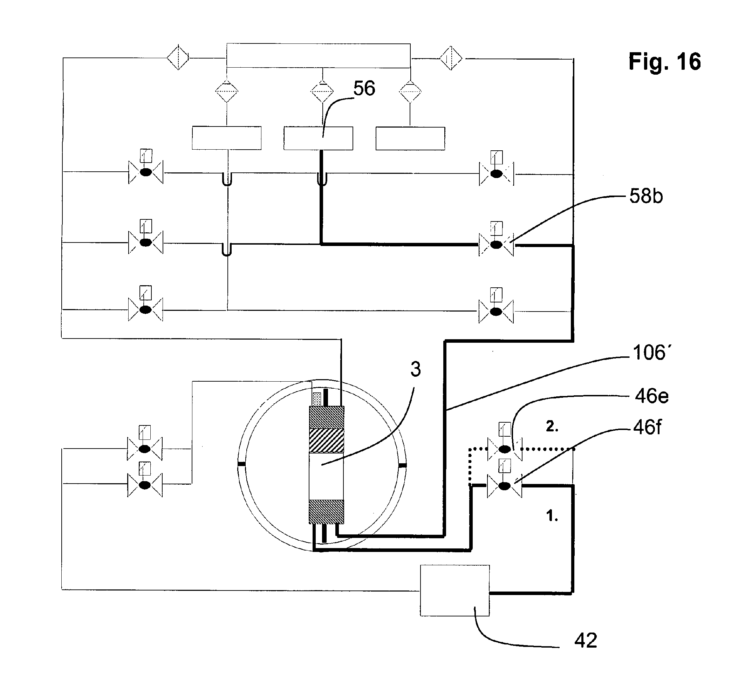

Figur 13 entspricht der Funktionalität von Figur 10 aber mit gedrehtem Reaktorgefäß. Dargestellt ist der Weg 100' der Nährlösung nach der Drehung des Reaktorgefäßes 3 um 180 Grad um die horizontale x-Drehachse bzw. z-Achse. Nun wird die Nährlösung analog zu dem Weg 100 in Figur 9, aber durch den Einlass 34 in das Reaktorgefäß 3 eingelassen und durch den Auslass 40 zum Abfallsammler 42 geleitet. Figur 14 zeigt den Weg 102' zum Abfallsammler 42. Auf gleiche Weise entspricht Figur 15 mit Weg 104' der Natronlauge der Figur 11 mit Weg 104 bei gedrehtem Reaktorgefäß 3. Figur 16 zeigt den Weg 106' der Pufferlösung bei gedrehtem Reaktorgefäß 32 analog zu Figur 12.

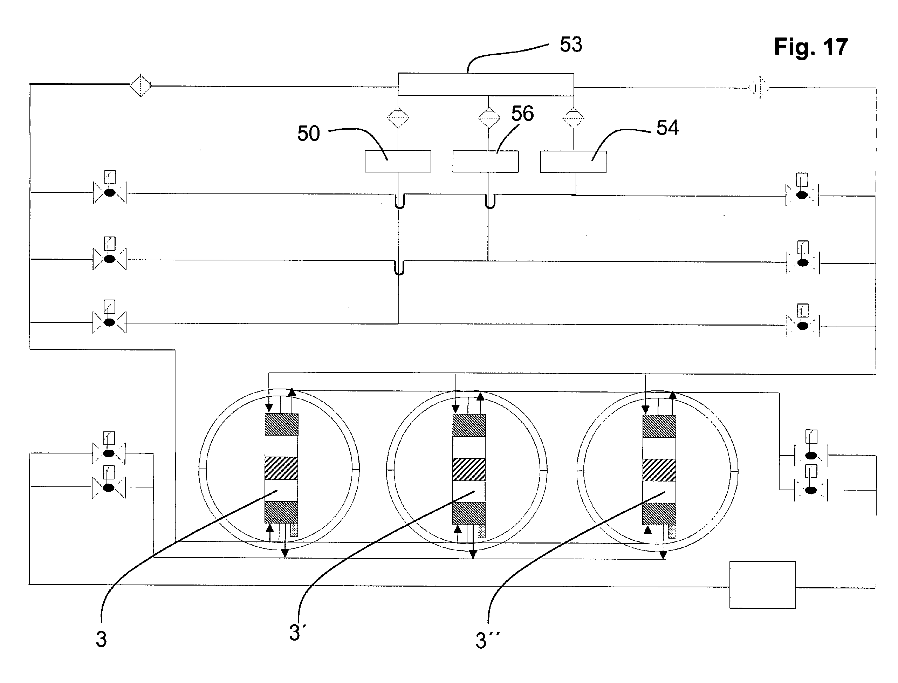

Die beschriebene Anordnung wird für kommerzielle Anwendung mit einer Vielzahl von parallel geschalteten Reaktorgefäße 3, 3', 3" verwendet, wie dies in Figur 17 dargestellt ist.

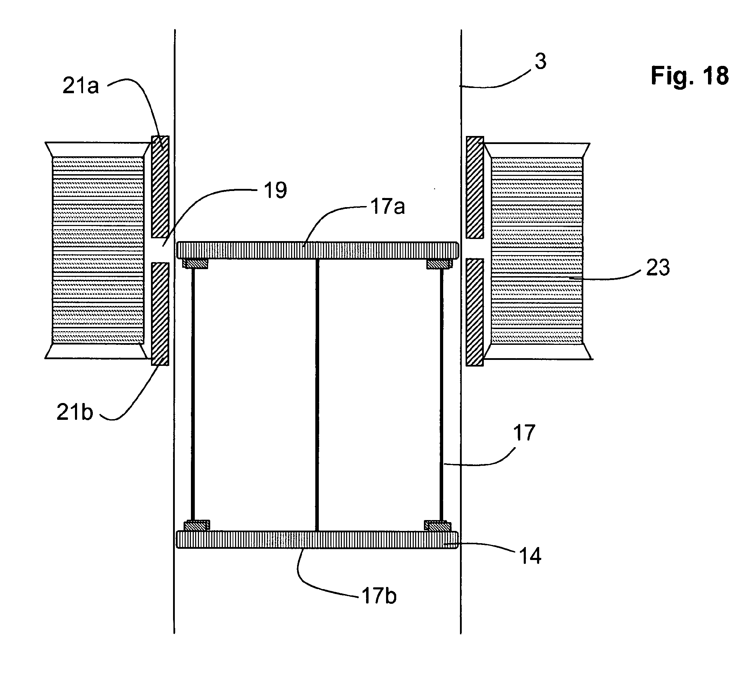

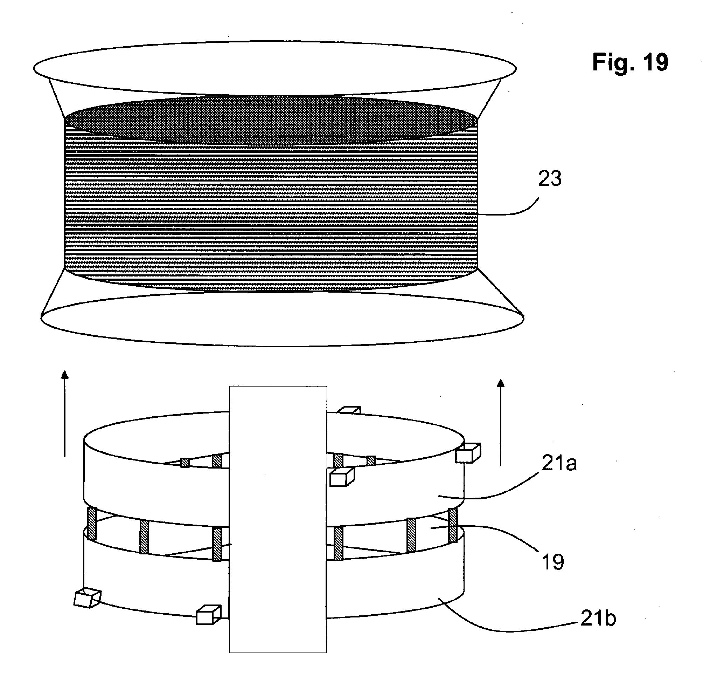

Die Arretierung der Zellträgereinheit 14 wird in Figur 18 und 19 beschrieben. Im Mittenbereich des Reaktorgefäßes 3 sind zwei Metallringe 21a und 21b vorgesehen. Zwischen den Metallringen 21a und 21b ist ein Luftspalt 19. Eine stromdurchflossene Spule 23 erzeugt ein Magnetfeld. Das Magnetfeld erstreckt sich durch den Luftspalt 19. Dort laufen die Magnetfeldlinien zusammen und arretieren die in diesem Bereich befindliche Metallplatte 17a der Zellträgereinheit.

Anhand der folgenden Beispiele sollen neue Verwendungsmöglichkeiten illustriert werden. Es versteht sich, dass diese nur beispielhaft aufgeführt sind, und sich auf alle möglichen Weisen verändern lassen, die sich dem Fachmann ohne weiteres erschließen.

### Ausführungsbeispiel 3: Kontinuierliche Fermentation von biologischen Zellen unter Verwendung eines Reaktors mit fixierter Materialträgereinheit

Für die kontinuierliche Langzeitkultivierung des Eukaryonten *Dictyostelium discoideum* wird ein geeignetes Medium bereitet:

### FM Medium (1 Liter)

250 ml 4x Aminosäuren
50 ml 20x Vitamine
20 ml 50x Salze
0.1 ml 10,000X Spurenelemente
10 g Glukose
0.87 g K₂HPO₄
50 mg Dihydrostreptomycinsulfat

Aus der mit *D. discoideum* inokulierten Ausgangskultur werden etwa 15 Erlenmeyerkolben angeimpft und bei 22°C für 24h bei 140 rpm geschüttelt. Der Fermenter wird vor dem Animpfen bei 121°C für 10 min. autoklaviert. Nach der Sterilisation auf eine geeignete Betriebstemperatur (Peltier-Elemente,Thermostat) werden über die Inox- Animpfstutzen 500 ml Animpfkulturen aus zehn bis zwölf Schüttelkolben in das Reaktionsgefäß gespritzt und die Anlagensteuerung (SPS) aktiviert. In der Kultivierungsphase führt das Reaktionsgefäß über seine horizontale Achse eine leichte Wippbewegung aus, um die kultivierten Zellen optimal mit Sauerstoff und Nährmedium zu versorgen. Nach etwa vier Stunden beginnen sich die Mikroorganismen zu vermehren. Die damit verbundene Trübung im Fermenter wird mittels Messungen der optischen Dichte (OD) quantifiziert, ab einem OD-Wert von ca. 1 wird auf kontinuierlichen Betrieb umgeschaltet. Die Verdünnungsrate wird so angepasst, dass eine maximale Zelldichte/Ausbeute resultiert. Zeigt die kontinuierliche Fermentation nach ca. drei bis vier Tagen stabile Parameter, wird mit einer In-situ-Extraktion begonnen. Der Fermentationsverlauf wird durch Messungen der optischen Dichte kontrolliert. Täglich werden automatisiert Fraktionen der gefilterten und ungefilterten Fermenterbrühe gezogen, analysiert und verwertet.

### Ausführungsbeispiel 4: Langzeitkultivierung von Gewebezelllinien zum Aufbau organoider Strukturen unter Verwendung eines Reaktors mit fixierter Materialträgereinheit

Für die Langzeitkultivierung der Zellen aus der 36. Passage der Keratinozyten- Zelllinie HaCaT (Human adult low Calcium high Temperature) wird Dulbecco's Modified Eagle Medium (DMEM) verwendet, welches einen Glukoseanteil von 4500 mg/l, Natriumpyruvat (110 mg/l), Phenolrot und Pyridoxin enthält. Unter sterilen Bedingungen wird das Medium mit 5 ml einer Gentamicin (5 mg)- L-Glutamin (200 mM)- Lösung und 56 ml Fötalem Kälberserum komplementiert.

Für die Subkultivierung der Zellen werden die HaCaT- Keratinozyten in einer Zelldichte von ca. 1x10⁶ Zellen /ml Komplettmedium in 5 Gewebekulturflaschen (75 cm²) mit Schräghals ausgesät und bei 37°C, 5% CO₂- Begasung sowie einer Luftfeuchtigkeit von 100% unter Lichtausschluss für 5 Tage inkubiert, bis eine 100%ige Konfluenz unter dem Lichtmikroskop sichtbar ist. Die Polypropylenlamellen (unwoven) der Zellträgereinheit werden mit einer 1,2%igen Alginatlösung (Keltone LV, Fa.Kelco) bestrichen und mit einer 102 mM CaCl₂ Lösung auf dem Zellträger ausgehärtet und fixiert. Der beschichtete Zellträger wird unter sterilen Bedingungen in den Reaktor eingeführt und DMEM als Nährmedium eingeleitet. Die Zellen werden trypsiniert (3 ml Trypsin/ EDTA 0,05%/ 0,02%) und auf die mit Alginat beschichtete Zellträgermatrix des Reaktionsgefäßes in DMEM aufgebracht. Nach 2 h sind die Zellen auf der Alginatschicht sedimentiert und adhärent. Die Langzeitkultivierung erfolgt bei 37°C, 5% CO₂- Begasung sowie einer Luftfeuchtigkeit von 100% und unter Lichtausschluss. In der Kultivierungsphase führt das Reaktionsgefäß über seine horizontale Achse eine leichte Wippbewegung aus, um die kultivierten Zellen optimal mit Sauerstoff und Nährmedium zu versorgen. Nach 2-3 Wochen sind die Lamellen des Zellträgers vollständig bewachsen, erste Differenzierungen der Keratinozyten und dreidimensionales Wachstum sind zu beobachten. Zum schonenden Abtrennen der Zellschichten von der synthetischen Matrix wird das Reaktionsgefäß über die integrierten Peltier-Elemente auf 4°C heruntergekühlt und das Kulturmedium abgelassen. Anschließend werden 55 mM Natriumcitrat und 0,15 mM Natriumchlorid (Fisher Scientific) bei einem pH von 6,8 in das Reaktionsgefäß eingelassen, bis die Alginatschicht eine Viskosität erreicht, die dazu führt, das sich Alginat und Zellverband von der synthetischen Zellträgermatrix löst und schonend aus dem Reaktionsgefäß entnommen werden kann. Nach entsprechender antibiotischer Behandlung steht die organoide Struktur für medizinische Anwendungen (z.B. als Transplantat im tissue-engineering) zur Verfügung.

### Ausführungsbeispiel 5: Isolierung und Kultivierung von neuen Ganzzell-Katalysatoren für die industrielle Prozessführung unter Verwendung eines Reaktors mit beweglicher Materialträgereinheit .

Mikroorganismen (hier: Archae) mit wirtschaftlich verwertbaren Eigenschaften befinden sich oft an extremen Standorten, so dass das Verfahren bereits mit der Probenentnahme im natürlichen Lebensraum der Zellen beginnt. In 3000 m Wassertiefe werden im Turtle-Pits-Field (Mittelatlantischer Rücken, 5°S) von einem Tauchroboter 50 mg Lavagestein aus einem hydrothermalen Schlot entnommen und im originären Umweltmedium (hier: Meereswasser) bei 4°C bis zur Aufarbeitung gekühlt. Mikroorganismen besiedeln das Substrat in Form von Konsortien (mikrobiellen Lebensgemeinschaften) und können daher nur in dieser Form in Kultur gebracht werden. Das Substrat wird in der Zellträgereinheit im Bioreaktor fixiert und bei einer Verdünnungsrate von D=0,21 zunächst im originären Meereswasser kultiviert. Ausgehend von den originären Umweltbedingungen (pH Wert, Temperatur, Salinität) werden die chemischen und physikalischen Parameter allmählich verändert und das Wachstum der (frei schwimmenden) Zellen per Dichtemessung erfasst. Die Archaeenpopulation wird bei einer Temperatur von 90°C kultiviert, um die Teilungsaktivität der thermophilen Mikroorganismen sicher zu stellen. Diese Temperatur wird graduell bis auf 70°C gesenkt, ohne die Teilungsaktivität zu beeinträchtigen. Wird im Reaktor bei 90°C eine optische Dichte von 1,5 erreicht, erfolgt automatisiert die Absenkung der Temperatur auf 89,5°C, bis die Zellen adaptiert sind und Zellteilung durch eine OD von 1,5 erneut sichtbar wird. Dieser Vorgang wiederholt sich, bis diese den Zielvorstellungen (= Prozessführungsbedingungen) entsprechen (= Turbidostat-Regime). Das Bioreaktorsystem bietet die für diesen Vorgang zwingend nötige Form der Langzeitkultivierung und ermöglicht so die schrittweise Adaptation.

### Ausführungsbeispiel 6: Optimierung von Ganzzell-Katalysatoren für Vermarkter biokatalytischer Verfahren unter Verwendung eines Reaktors mit beweglicher Materialträgereinheit

Zellen, die zuvor molekularbiologisch verändert wurden und eine schlechte Ausbeute oder instabiles Wachstum aufweisen, werden stabilisiert und zu einer gut wachsenden Zellkultur aufgebaut. Das Bioreaktorsystem wird mit dem Ursprungsmedium des Auftraggebers betrieben. Die zu optimierenden Ganzzell-Katalysatoren des Auftraggebers werden eingebracht und die Parameter im Turbidostat-Regime graduell angehoben, bis adäquate Generationszeiten und robustes Wachstum erreicht sind.

Das Bakterium β1308 (Abkömmling von *E*. *coli* MG1655, in dem die Kodons 26 bis 256 des thyA Gens durch das erm Gen von Tn1545 ersetzt wurden), soll ohne externe Schwefelquelle (hier: K₂SO₄) und ohne externe Stickstoffquelle (hier: NH₄Cl₂) im Medium auskommen, um den Produktionsprozess wirtschaftlich effizienter zu gestalten. Da β1308 auf Schwefel und Stickstoff angewiesen ist, soll das Bakterium beide Substanzen aus der Aminosäure Methionin beziehen, die sowohl Stickstoff, als auch Schwefel enthält. Zunächst wird β1308 bei 37°C in einer Übernachtkultur in AC-Medium kultiviert:
**A+C Medium**
Zitronensäure 4 mM
MgCl₂ x 6 H₂O 1 mM
NH₄Cl₂ 14 mM
K₂HPO₄ 38 mM
FeCl₃ (10mM) 1 ml
Mannit 11 mM
Thymin 1 mM
Methionin 5 mM
K₂SO₄ 1 mM

Das Reaktionsgefäß wird mit allen Anschlüssen bei 121°C autoklaviert und nach dem Abkühlen mit A+C Medium in Betrieb genommen. Die Verdünnungsrate sollte in diesem Kultivierungsstadium nicht größer als 0,5/d sein. Dieses Regime wird über 14 Tage aufrechterhalten, damit sich ein robuster Biofilm entwickeln kann. Über eine Infrarot-Gabellichtschranke (Transmission) wird kontinuierlich die Biomasse der planktischen Zellen außerhalb des Biofilms erfasst und kann nach Eichung auf die optische Dichte (OD 600nm) zurückgerechnet werden. Übersteigt die optische Dichte einen Wert von 1,4 wird automatisiert ein zweites (permissives) Nährmedium eingeleitet, welches keine Schwefelquelle mehr enthält:
**A+B-Medium**
Zitronensäure 4 mM
MgCl₂ x 6 H₂O 1 mM
NH₄CL 14 mM
K₂HPO₄ 38 mM
FeCl₃ (10mM) 1 ml
Mannit 11 mM
Thymin 1 mM
Methionin 5 mM

Das Bakterium β1308 adaptiert allmählich an das neue Medium, indem das Erreichen einer hohen Zelldichte die Einleitung des permissiven Mediums zu Folge hat. Erreicht β1308 zuverlässig OD 1,4 bei D=0,5 wird das A+C Medium ersetzt durch J-Medium (keine N- und S-Quelle außerhalb von Methionin). AB Medium stellt das nicht permissive, J Medium das permissive Medium dar:
**J-Medium**
Zitronensäure 4 mM
MgCl₂ x 6 H₂O 1 mM
K₂HPO₄ 38 mM
FeCl₃(10mM) 1 ml
Mannit 11 mM
Thymin 1 mM
Methionin 5 mM

Analog zum Turbidostat-Verfahren mit AB Medium erfolgt die Adapatation von β1308 an das J Medium. Nur Varianten, die sowohl Stickstoff, als auch Schwefel aus Methionin gewinnen können, überleben das Regime. Das Wachstum der vom Biofilm generierten planktischen Zellen wird erfasst. Erreicht β1308 zuverlässig OD 1,4 bei D=0,5 werden die Zellen down-stream unter sterilen Bedingungen entnommen und auf das Vorhandensein des erm Gen von Tn1545 (Sequenz hier als Stellvertreter für andere molekularbiologische Veränderungen) hin überprüft.

### Ausführungsbeispiel 7: Atomlagenabscheidung als Werkzeug der Nanotechnologie unter Verwendung eines Reaktors mit beweglicher Materialträgereinheit

In die Abscheidungskammer mit einem Volumen von max. 25 1 wird das zu beschichtende Substrat mit einer maximalen Oberfläche von 300 nm eingebracht und ist hier durch die Magnetarretierung in jeder Phase des Beschichtungszyklus steuerbar. Um die Kondensierung der Precusoren zu verhindern, werden die Reaktorwände über den gesamten Zeitraum auf eine Temperatur von 150° C erhitzt. Ausgehend von dem Zulauf 36 (Blasdüsenrohr) wird in allen Leitungssystemen in diesem Areal eine Temperatur von 150°C und im Bereich der Abscheidungskammer eine Temperatur von 134° gewährleistet. Die Beweglichkeit des Substrats ermöglicht die optimale Positionierung im Gasstrom und reduziert das Reaktionsvolumen insgesamt. Es empfiehlt sich nicht, den Abstand zwischen Substrat und Gaseinleitung größer als 1 mm zu wählen, die Temperaturen sollten nicht unter 200°C und nicht über 350°C liegen. Der Kammerdruck wird durch zwei Pumpen aufrecht erhalten, welche bei einem Druck von 10⁻⁴ Pa eine Förderleistung von 100 1/s, bzw. 20 1/s erbringen. Für die thermale Beschichtung auf Ta-N Basis wird als Precusor TBTDET (Epichem 99,99%) eingesetzt, der bei einer Temperatur von 80°C in die Kammer eingeleitet wird. Im Zyklus wird zunächst TBTDET mit 70 sccm Argon als Trägergas für eine Sekunde eingeleitet. Dann werden die Reaktionsprodukte mit 1000sccm Argon aus der Abscheidungskammer gespült und mit 50 sccm NH₃ über 3s und 1000sccm Ar für 2 s gespült, um das Reaktionsgefäß auf den nächsten Zyklus vorzubereiten.

### Bezugszeichenliste

- 1: erstes Chassis
- 2: zweites Chassis
- 3,3',3": Reaktorgefäß
- 4: Materialträger oder Matrix (z.B. Substrat für biologische Zellen)
- 5/7: Achsen für vertikale Rotation
- 6 / 8: Achsen für horizontale Rotation
- 38/ 40: Abläufe
- 34/ 36: Zuläufe
- 13: Fixierungen für Matrix
- 14: Materialträgereinheit
- 16: Steg
- 17a: obere Platte
- 17b: untere Platte
- 18: Steg
- 19: Luftspalt
- 20: Steg
- 21 a, b: Metallringe
- 22: Hohlraum
- 23: stromdurchflossene Spule
- 24: Chassis für Rotation in der z-Ebene
- 25: Kupplung
- 26: Unterseite der oberen Platte 17a
- 27: Distanzbolzen
- 28: Oberseite der unteren Platte 17b
- 29: Montagewand
- 30: Schienen
- 31: Kugellager
- 32: Flüssigkeit
- 33: Mantellänge des Reaktorgefäßes
- 35: horizontale Ebene
- 39: erster Elektromagnet
- 42: Abfallsammler
- 43: Welle des Elektromotors 45c
- 44: gemeinsame Zuleitung für die Einlässe 34, 36
- 45a,b,c: Elektro- oder Schrittmotor
- 46a, b: Ventile zur Steuerung des Zuflusses des Nährmediums
- 46 c-f: Ventile zur Steuerung des Ablaufs des Nährmediums
- 47: Chassis für Elektromotor 45c
- 48: Antriebsritzel
- 49: zweiter schaltbarer Elektromagnet
- 50: Reservoir für Reaktionsmedium, insbesondere Nährmedium
- 51: Ein- und Auslassanschluss für organische und/oder anorganische Substanzen
- 53: Reservoir für eine Gasquelle
- 54: Reservoir für Natronlauge
- 55: Zahnrad an Chassis 1
- 56: Reservoir für Pufferlösung
- 57: Schrittmotor für höherer Last
- 58a, b: Ventile für Zulass der Pufferlösung
- 59: Antriebswelle für Schrittmotor 57
- 59a: Antriebswellenlager
- 60 a, b: Ventile für Zulass der Natronlauge
- 100 / 100': Zulaufweg des Nährmediums
- 102 / 102': Ablaufweg des Nährmediums
- 104/104': Zulaufweg der Natronlauge
- 106 / 106': Zulaufweg der Pufferlösung

## Patentansprüche

1. Reaktorsystem umfassend mindestens ein Reaktorgefäß zur Herstellung und/oder Behandlung eines Materials, insbesondere zur kontinuierlichen Kultivierung von biologischem Material,
**dadurch gekennzeichnet, dass**
das Reaktorgefäß (3) um seine drei räumlichen (x, y, z)-Achsen drehbar angeordnet ist, wobei das Reaktorgefäß an mindestens drei Chassis (1, 2, 24) gekoppelt ist, und mindestens eine innerhalb des Reaktorgefäßes (3) angeordnete Materialträgereinheit (14) aufweist.

2. Reaktorsystem nach Anspruch 1, **dadurch gekennzeichnet, dass** die Materialträgereinheit (14) im Reaktorgefäß (3) fixiert angeordnet ist.

3. Reaktorsystem nach Anspruch 1 oder 2, **dadurch gekennzeichnet, dass** die Materialträgereinheit (14) drei dimensional, insbesondere vertikal im Reaktorgefäß (3) beweglich ist.

4. Reaktorsystem nach Anspruch 3, **dadurch gekennzeichnet, dass** die Materialträgereinheit (14) im Reaktorgefäß (3) von außen in mindestens zwei unterschiedlichen Positionen im Reaktorgefäß (3) lösbar arretierbar ist.

5. Reaktorsystem nach einem der Ansprüche 3 oder 4, **dadurch gekennzeichnet, dass** die Materialträgereinheit (14) nach Drehung des Reaktorgefäßes (3) um mindestens eine der drei räumlichen Achsen, insbesondere um die horizontale x- Achse, im jeweils oberhalb der mindestens einen der drei räumlichen Achsen, insbesondere der horizontalen Achse gelegenen Abschnitt des Reaktorgefäßes (14) arretierbar ist.

6. Reaktorsystem nach einem der vorhergehenden Ansprüche, **dadurch gekennzeichnet, dass** der jeweils unterhalb der arretierten Materialträgereinheit (14) befindliche Teil des Reaktorgefäßes (3) mit Anschlüssen versehen (34, 36, 38, 40) ist, über welche das Reaktorgefäß (3) mit einer Gasquelle (53) oder Quellen für Reaktions-, Reinigungs- und Pufferlösung (50, 54, 56), verbindbar ist.

7. Reaktorsystem nach einem der vorhergehenden Ansprüche, **dadurch gekennzeichnet, dass** das Reaktorgefäß (3) mit einem oder mehreren Anschlüssen (34, 36, 38, 40) versehen ist, über welche Flüssigkeiten und/oder Gase durch das Reaktorgefäß (3) hindurchleitbar sind.

8. Reaktorsystem nach einem der vorgehenden Ansprüche, **dadurch gekennzeichnet, dass** das Reaktorgefäß (3) von einem zylinderförmigen Gefäß gebildet ist, der in seinem mittleren Bereich zumindest an einer Achse drehbeweglich aufgehängt ist.

9. Reaktorsystem nach einem der vorgehenden Ansprüche, **gekennzeichnet durch** Mittel zum Auslösen einer Drehbewegung des Reaktorgefäßes (3) um 180° um die horizontale Achse in auswählbaren Intervallen.

10. Materialträgereinheit (14) zur Verwendung in einem Reaktorsystem nach einem der vorhergehenden Ansprüche umfassend mindestens einen Materialträger (4), insbesondere einen Materialträger (4) dessen Oberfläche mit Lamellen versehen ist.

11. Materialträgereinheit nach Anspruch 10, **dadurch gekennzeichnet, dass** der Materialträger (4) ein flächiger Zellträger ist.

12. Verwendung von einem Reaktorsystem nach einem der vorgehenden Ansprüche zur Kultivierung von biologischem Material, insbesondere Enzymen, Zellen, Biofilmen oder Geweben und zur Herstellung von Produkten unter kontrollierten, sterilen und keimfreien Bedingungen, insbesondere im Bereich der Biologie, Pharmazie, Halbleitertechnologie, Optik, Lack- und Farbindustrie.

13. Verfahren zur Herstellung von Produkten in einem Reaktorsystem nach einem der Ansprüche 1 bis 9 mit einem um mindestens eine der drei (x, y, z) Achsen drehbaren Reaktorgefäß in dem eine Materialträgereinheit angeordnet ist, **gekennzeichnet durch** die Schritte
(a) Abscheiden oder Auftragen einer Zusammensetzung auf der Materialträgereinheit (14);
(b) ggf. Arretieren der Materialträgereinheit (14) im jeweils oberhalb der horizontalen Achse gelegenen Abschnitt des Reaktorgefäßes (3);
(c) Drehen des Reaktorgefäßes (3);
(d) Reinigen des Bereichs in dem Reaktorgefäßes (3) unterhalb der Materialträgereinheit (14); und
(e) ggf. Lösen der Arretierung der Materialträgereinheit (14), so dass diese sich in den gereinigten Bereich in dem Reaktionsgefäß (3) bewegt.

14. Verfahren nach Anspruch 13 zur Kultivierung von biologischem Material, insbesondere von Zellen, Biofilmen und Geweben, **gekennzeichnet durch** die Schritte
(a) Kultivieren der Kultur auf der Materialträgereinheit (14);
(b) ggf Arretieren der Materialträgereinheit (14) im jeweils oberhalb der horizontalen Achse gelegenen Abschnitt des Reaktorgefäßes (3),
(c) Drehen des Reaktorgefäßes (3);
(d) Reinigen des Bereichs in dem Reaktorgefäß (3) unterhalb der Materialträgereinheit (14); und
(e) ggf. Lösen der Arretierung der Materialträgereinheit (14), so dass diese sich in den gereinigten Bereich in dem Reaktorgefäß (3) bewegt;

15. Verfahren nach einem der Ansprüche 13 oder 14, **dadurch gekennzeichnet, dass** die Schritte (a) bis (e) in regelmäßigen Zeitabständen wiederholt werden.

## Claims

1. A reactor system comprising at least one reactor vessel for the preparation and/or treatment of a material, in particular for the continuous cultivation of biological material,
**characterized in that**
the reactor vessel (3) is rotatably arranged about its three spatial (x, y, z) axes, wherein the reactor vessel is coupled to at least three chassis (1, 2, 24), and includes at least one material carrier unit (14) arranged within the reactor vessel (3).

2. The reactor system according to claim 1, **characterized in that** the material carrier unit (14) is fixed inside the reactor vessel (3).

3. The reactor system according to claim 1 or 2, **characterized in that** the material carrier unit (14) is three-dimensionally, in particular vertically movable in the reactor vessel (3).

4. The reactor system according to claim 3, **characterized in that** the material carrier unit (14) is releasably lockable in the reactor vessel (3) from outside in at least two different positions in the reactor vessel (3).

5. The reactor system according to any of claims 3 or 4, **characterized in that** after rotating the reactor vessel (3) about at least one of the three spatial axes, in particular about the horizontal x-axis, the material carrier unit (14) is lockable in the portion of the reactor vessel (3) each located above the at least one of the three spatial axes, in particular the horizontal axis.

6. The reactor system according to any of the preceding claims, **characterized in that** the part of the reactor vessel (3) each located below the locked material carrier unit (14) is provided with ports (34, 36, 38, 40) via which the reactor vessel (3) is connectable with a gas source (53) or sources for reaction, cleaning and buffer solution (50, 54, 56).

7. The reactor system according to any of the preceding claims, **characterized in that** the reactor vessel (3) is provided with one or more ports (34, 36, 38, 40) via which liquids and/or gases can be passed through the reactor vessel (3).

8. The reactor system according to any of the preceding claims, **characterized in that** the reactor vessel (3) is formed by a cylindrical vessel which in its middle region is rotatably suspended at least on one axis.

9. The reactor system according to any of the preceding claims, **characterized by** means for triggering a rotary movement of the reactor vessel (3) by 180° about the horizontal axis at selectable intervals.

10. A material carrier unit (14) for use in a reactor system according to any of the preceding claims, comprising at least one material carrier (4), in particular a material carrier (4) whose surface is provided with lamellas.

11. The material carrier unit according to claim 10, **characterized in that** the material carrier (4) is a flat cell carrier.

12. Use of a reactor system according to any of the preceding claims for cultivating biological material, in particular enzymes, cells, biofilms or tissues and for manufacturing products under controlled, sterile and aseptic conditions, in particular in the field of biology, pharmaceutics, semiconductor technology, optics, paint and coatings industry.

13. A method for manufacturing products in a reactor system according to any of claims 1 to 9 with a reactor vessel rotatable about at least one of the three (x, y, z) axes, in which a material carrier unit is arranged, **characterized by** the steps
(a) depositing or applying a composition on the material carrier unit (14);
(b) possibly locking the material carrier unit (14) in the portion of the reactor vessel (3) each located above the horizontal axis;
(c) rotating the reactor vessel (3);
(d) cleaning the region in the reactor vessel (3) below the material carrier unit (14); and
(e) possibly releasing the locking of the material carrier unit (14), so that the same moves into the cleaned region in the reaction vessel (3).

14. The method according to claim 13 for cultivating biological material, in particular cells, biofilms and tissues, **characterized by** the steps
(a) cultivating the culture on the material carrier unit (14);
(b) possibly locking the material carrier unit (14) in the portion of the reactor vessel (3) each located above the horizontal axis;
(c) rotating the reactor vessel (3);
(d) cleaning the region in the reactor vessel (3) below the material carrier unit (14); and
(e) possibly releasing the locking of the material carrier unit (14), so that the same moves into the cleaned region in the reactor vessel (3).

15. The method according to any of claims 13 or 14, **characterized in that** the steps (a) to (e) are repeated at regular time intervals.

## Revendications

1. Système de réacteur comprenant au moins un récipient réacteur destiné à la production et/ou au traitement d'une matière, en particulier à la culture continue de matière biologique,
**caractérisé en ce que**
le récipient réacteur (3) est disposé pour pouvoir tourner autour de ses trois axes tridimensionnels (x, y, z), le récipient réacteur étant couplé à au moins trois châssis (1, 2, 24) et comportant au moins une unité porteuse de matière (14) disposée à l'intérieur du récipient réacteur (3).

2. Système de réacteur selon la revendication 1, **caractérisé en ce que** l'unité porteuse de matière (14) est disposée en étant fixée dans le récipient réacteur (3).

3. Système de réacteur selon la revendication 1 ou 2, **caractérisé en ce que** l'unité porteuse de matière (14) est mobile dans les trois dimensions, en particulier verticalement dans le récipient réacteur (3).

4. Système de réacteur selon la revendication 3, **caractérisé en ce que** l'unité porteuse de matière (14) dans le récipient réacteur (3) peut être réversiblement bloquée de l'extérieur en au moins deux différentes positions dans le récipient réacteur (3).

5. Système de réacteur selon la revendication 3 ou 4, **caractérisé en ce qu'**après rotation du récipient réacteur (3) autour d'au moins l'un des trois axes tridimensionnels, en particulier autour de l'axe horizontal x, l'unité porteuse de matière (14) peut être bloquée dans la section du récipient réacteur (3) se trouvant chaque fois au-dessus dudit au moins l'un des trois axes tridimensionnels, en particulier de l'axe horizontal.

6. Système de réacteur selon l'une quelconque des revendications précédentes, **caractérisé en ce que** la partie du récipient réacteur (3) qui se trouve chaque fois au-dessous de l'unité porteuse de matière (14) bloquée est munie de raccords (34, 36, 38, 40) au moyen desquels le récipient réacteur (3) peut être raccordé à une source de gaz (53) ou à des sources de solution réactionnelle, de nettoyage et tampon (50, 54, 56).

7. Système de réacteur selon l'une quelconque des revendications précédentes, **caractérisé en ce que** le récipient réacteur (3) est muni d'un ou plusieurs raccords (34, 36, 38, 40) au moyen desquels il est possible de faire passer des liquides et/ou des gaz dans le récipient réacteur (3).

8. Système de réacteur selon l'une quelconque des revendications précédentes, **caractérisé en ce que** le récipient réacteur (3) est constitué d'un récipient cylindrique qui est accroché dans sa zone médiane, de façon à pouvoir tourner, au moins à un axe.

9. Système de réacteur selon l'une quelconque des revendications précédentes, **caractérisé par** des moyens destinés à déclencher à intervalles pouvant être choisis un mouvement rotatif du récipient réacteur (3) de 180° autour de l'axe horizontal.

10. Unité porteuse de matière (14) destinée à l'utilisation dans un système de réacteur selon l'une quelconque des revendications précédentes, comprenant au moins un support de matière (4), en particulier un support de matière (4) dont la surface est munie de lamelles.

11. Unité porteuse de matière selon la revendication 10, **caractérisée en ce que** le support de matière (4) est un support de cellules plat.

12. Utilisation d'un système de réacteur selon l'une quelconque des revendications précédentes, pour la culture de matière biologique, en particulier d'enzymes, de cellules, de biofilms ou de tissus et pour l'élaboration de produits dans des conditions contrôlées, stériles et exemptes de germes, notamment dans le domaine de la biologie, la pharmacie, la technologie des semi-conducteurs, l'optique, l'industrie des peintures et vernis.

13. Procédé pour l'élaboration de produits dans un système de réacteur selon l'une quelconque des revendications 1 à 9, comportant un récipient réacteur pouvant tourner autour d'au moins l'un des trois axes (x, y, z), dans lequel est disposée une unité porteuse de matière, **caractérisé par** les étapes
(a) dépôt ou application d'une composition sur l'unité porteuse de matière (14) ;
(b) éventuellement blocage de l'unité porteuse de matière (14) dans la section du récipient réacteur (3) qui se trouve chaque fois au-dessus de l'axe horizontal ;
(c) mise en rotation du récipient réacteur (3) ;
(d) nettoyage de la zone dans le récipient réacteur (3) au-dessous de l'unité porteuse de matière (14) ; et
(e) éventuellement déblocage de l'unité porteuse de matière (14), de sorte que cette dernière se meut dans la zone nettoyée dans le récipient réacteur (3).

14. Procédé selon la revendication 13 pour la culture de matière biologique, en particulier de cellules, biofilms et tissus, **caractérisé par** les étapes
(a) culture de la matière biologique cultivée sur l'unité porteuse de matière (14) ;
(b) éventuellement blocage de l'unité porteuse de matière (14) dans la section du récipient réacteur (3) qui se trouve chaque fois au-dessus de l'axe horizontal ;
(c) mise en rotation du récipient réacteur (3) ;
(d) nettoyage de la zone dans le récipient réacteur (3) au-dessous de l'unité porteuse de matière (14) ; et
(e) éventuellement déblocage de l'unité porteuse de matière (14), de sorte que cette dernière se meut dans la zone nettoyée dans le récipient réacteur (3).

15. Procédé selon la revendication 13 ou 14, **caractérisé en ce qu'**on répète à intervalles de temps réguliers les étapes (a) à (e).
